# EUROPEAN PATENT APPLICATION

(11) **EP 4 454 695 A1**
(43) Date of publication of application: **30.10.2024**
(21) Application number: 22911382.4
(22) Date of filing: 23.12.2022
(51) Int. Cl.: A61M 25/10, A61B 18/04

(54) **BALLOON CATHETER AND BALLOON CATHETER SYSTEM**

(30) Priority: 24.12.2021 JP 2021211537
(71) Applicant: Toray Industries, Inc., Tokyo 103-8666 (JP)
(72) Inventor: HARADA Hiroyuki, Tokyo 103-8666 (JP); TSUKAMOTO Kota, Tokyo 103-8666 (JP); TAKAOKA Motoki, Osaka-shi, Osaka 530-8222 (JP); TANAHASHI Akio, Otsu-shi, Shiga 520-2141 (JP); MIYASHITA Masaomi, Otsu-shi, Shiga 520-8558 (JP)
(74) Representative: Kador & Partner Part mbB
(86) International application number: PCT/JP2022/047504
(87) International publication number: WO 2023/120680

(57) **Abstract**

A balloon catheter (15) includes a balloon (25), a catheter shaft (28), a heating unit (39), and an electrode temperature sensor (45). The catheter shaft (28) has an outer cylinder shaft (30) connected to the proximal end (25a) of the balloon (25), and an inner cylinder shaft (35) extending into the balloon (25) to be connected to the distal end (25a) of the balloon (25). The inner cylinder shaft (35) passes inside the outer cylinder shaft (30). The gap between the inner cylinder shaft (35) and the outer cylinder shaft (30) defines a liquid flow path (LP) leading to the inner space of the balloon (25). The heating unit (39) includes multiple heating electrodes (40). The multiple heating electrodes (40) are distributed over the outer circumferential surface of the inner cylinder shaft (35) in the balloon (25). The electrode temperature sensor (40) is provided in the balloon (25) to acquire information on the temperature of the heating electrodes (40).

## Description

### Technical Field

The present invention relates to a balloon catheter and a balloon catheter system.

### Background Art

Catheter ablation therapy is a method for treating a patient by using a catheter inserted into the patient's body to ablate a target site in the body. For example, the target site is destroyed by ablation to treat diseases such as arrhythmia due to atrial fibrillation, endometriosis, cancer, etc. As disclosed in JP 3611799 B2 and JP 4747141 B2, as a catheter used for the catheter ablation therapy, a balloon catheter having a balloon at the distal end thereof is known.

When the balloon catheter is inserted into the body, the balloon is deflated and stretched in the longitudinal direction of the balloon catheter. Then, the balloon catheter inserted into the body is supplied with a liquid to inflate the balloon. The temperature of the liquid in the balloon is controlled, thereby the surface temperature of the balloon is controlled. By bringing the balloon whose surface temperature has been controlled to a predetermined temperature into contact with a circular target site, for example, a connection area of a vein to the atrium, the circumferential target side can be ablated at once.

The surface temperature of the balloon is controlled by heating the liquid in the balloon by means of a heating electrode located inside the balloon and a counter electrode (return pad) located outside the balloon, by agitating the liquid in the balloon to diffuse the heated liquid, and by controlling the input to the heating electrode. In order to heat the liquid in the balloon, high-frequency current conduction (high-frequency energization) is performed to the heating electrode to pass a high-frequency current between the heating electrode and the counter electrode, so that the liquid in the balloon generates Joule heat. Thus, by controlling the high-frequency current conduction to the heating electrode, it is possible to control the heating of the liquid in the balloon. Note that simply heating of the liquid in the balloon by using the heating electrode cannot increase the surface temperature of the balloon up to a predetermined surface temperature, because a temperature gradient in the liquid in the balloon is generated around the heating electrode. Thus, the liquid in the balloon is agitated to diffuse the heated liquid in order to increase the surface temperature of the balloon up to a predetermined surface temperature.

WO 2021/201078 A1 teaches a heating electrode equipped with a temperature sensor to control the high-frequency current conduction to the heating electrode based on information acquired by the temperature sensor. However, depending on the angle at which the balloon catheter is pressed against a target site, it is sometimes impossible to increase the surface temperature of the balloon up to a predetermined temperature even by agitating the liquid in the balloon.

### Disclosure of The Invention

The present invention has been made in view of the above points. The object of the present invention is to control the surface temperature of the balloon in a reliable manner.

A balloon catheter of the present invention comprises a balloon, a catheter shaft, a heating unit, and an electrode temperature sensor, wherein: the catheter shaft has an outer cylinder shaft connected to a proximal end of the balloon, and an inner cylinder shaft extending into the balloon to be connected to a distal end of the balloon, the inner cylinder shaft passes inside the outer cylinder shaft, and a gap between the inner cylinder shaft and the outer cylinder shaft defines a liquid flow path leading to an inner space of the ballon; the heating unit includes multiple heating electrodes; the multiple heating electrodes are distributed over an outer circumferential surface of the inner cylinder shaft in the balloon; and the electrode temperature sensor is provided in the balloon to acquire information on a temperature of the heating electrodes.

In the balloon catheter of the present invention, the heating electrodes may be coil electrodes that are formed by winding a single lead wire around the inner cylinder shaft at multiple locations thereon.

In the balloon catheter of the present invention, a length of an area in which the heating electrodes are located may be half or more than half a length of the inner cylinder shaft in the balloon.

In the balloon catheter of the present invention, the number of the heating electrodes may be between 2 and 6.

In the balloon catheter of the present invention, a length of the heating electrode may be 3 mm or more.

In the balloon catheter of the present invention, a length between the heating electrode and the heating electrode adjacent thereto may be between 2 and 14 mm.

In the balloon catheter of the present invention, a distance between a proximal end of the multiple heating electrodes and a distal end of the outer cylinder shaft may be between 0 and 5 mm.

The balloon catheter of the present invention may comprise a liquid-flow-path temperature sensor provided in the liquid flow path to acquire information on a temperature of a liquid flowing in the liquid flow path, wherein the distance between the proximal end of the multiple heating electrodes and the distal end of the outer cylinder shaft may be between 3 and 5 mm.

A balloon catheter system of the present invention comprises: the aforementioned balloon catheter; a supply unit that supplies a liquid to the liquid flow path; an agitator that agitates the liquid in the balloon by repeatedly supplying the liquid to the liquid flow path and discharging the liquid from the liquid flow path; and a control unit electrically connected to the heating electrodes to provide the heating electrodes with electric energy; wherein the control unit provides the heating electrodes with electric energy based on information on a temperature acquired by the electrode temperature sensor.

In the balloon catheter system of the present invention, the balloon catheter may have a liquid-flow-path temperature sensor provided in the liquid flow path to acquire information on a temperature of a liquid flowing in the liquid flow path; the distance between the proximal end of the multiple heating electrodes and the distal end of the outer cylinder shaft may be between 3 and 5 mm; and a distance from the distal end of the outer cylinder shaft up to the liquid-flow-path temperature sensor may be no more than a value obtained by dividing an amount of the liquid discharged by the agitator from the liquid flow path by a cross-sectional area of the liquid flow path.

The present invention can control the surface temperature of the balloon in a reliable manner.

### Brief Description of the Drawings

Fig. 1 is a view describing an embodiment and showing a balloon catheter system and a balloon catheter.
Fig. 2 is a view showing a distal end portion of the balloon catheter of Fig. 1, with a balloon being inflated.
Fig. 3 is a view, to an enlarged scale, showing a part of the distal end portion shown in Fig. 2.
Fig. 4 is a view showing the distal end portion of the balloon catheter of Fig. 1, with the balloon being deflated and stretched.
Fig. 5 is a sectional view along a I-I line of Fig. 2.
Fig. 6 is a sectional view along a II-II line of Fig. 2.
Fig. 7 is a view showing the distal end portion of the balloon, for describing flow of a liquid when a liquid is discharged from a liquid flow path into the balloon.
Fig. 8 is a view showing the distal end portion of the balloon, for describing flow of the liquid when a liquid is suctioned from inside the balloon to the liquid flow path.
Fig. 9 is a view showing a liquid temperature distribution in the distal end portion of the balloon catheter in a coaxial state, which is obtained by thermo-fluid analysis using CAE.
Fig. 10 is a view showing a liquid temperature distribution in the distal end portion of the balloon catheter in a non-coaxial state, which is obtained by thermos-fluid analysis using CAE.
Fig. 11 is a view showing the distal end portion of the balloon catheter shown in Fig. 2, for describing flow of the liquid supplied from the liquid flow path into the balloon, with the catheter being pressed hard against a treatment site.
Fig. 12 is a view corresponding to Fig. 2, for describing a modification example of the balloon catheter.
Fig. 13 is a view corresponding to Fig. 3 and showing, to an enlarged scale, a part of the distal end portion shown in Fig. 12.
Fig. 14 is a view corresponding to Fig. 2, for describing another modification example of the balloon catheter.
Fig. 15 is a view corresponding to Fig. 3 and showing, to an enlarged scale, a part of the distal end portion shown in Fig. 14.
Fig. 16 is a view corresponding to Fig. 2 and showing a distal end portion of a conventional balloon catheter.
Fig. 17 is a view for describing the flow of the liquid in the balloon of the balloon catheter shown in Fig. 16 when the balloon is pressed hard against a treatment site and a liquid is supplied from a liquid flow path into the balloon.
Fig. 18 is a view for describing the flow of the liquid in the balloon of the balloon catheter shown in Fig. 16 when the balloon is pressed hard against a treatment site and a liquid is suctioned from inside the balloon to the liquid flow path.
Fig. 19 is a graph showing temperature differences between estimated surface temperatures of the balloon and actual surface temperatures thereof, for each distance between a heating electrode and an outer cylinder shaft.
Fig. 20 is a view for describing an experiment method using the balloon catheter system.
Fig. 21 is a table showing results obtained from the experiment shown in Fig. 20.

### Mode for Carrying out the Invention

One embodiment of the present invention is described below with reference to the drawings. In the drawings attached to the specification, a scale dimension, an aspect ratio and so on are changed and exaggerated from the actual ones, for the convenience of easiness in illustration and understanding. In addition, terms used herein to specify shapes, geometric conditions and their degrees, e.g., "parallel", "orthogonal", "same", etc., and values of a length and an angle are not limited to their strict definitions, but construed to include a range capable of exerting a similar function.

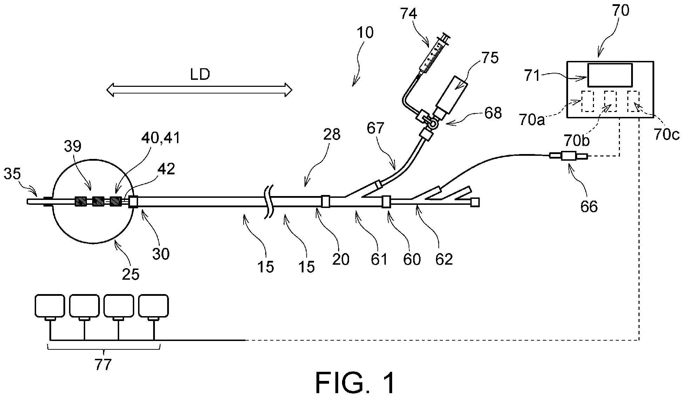

A balloon catheter system 10 shown in Fig. 1 has a balloon catheter 15, a control unit 70 connected to the balloon catheter 15, a supply unit 74, and an agitator 75. In addition, the balloon catheter 15 has a catheter body 20 having a longitudinal direction LD, and a handle 60 connected to a proximal end of the catheter body 20.

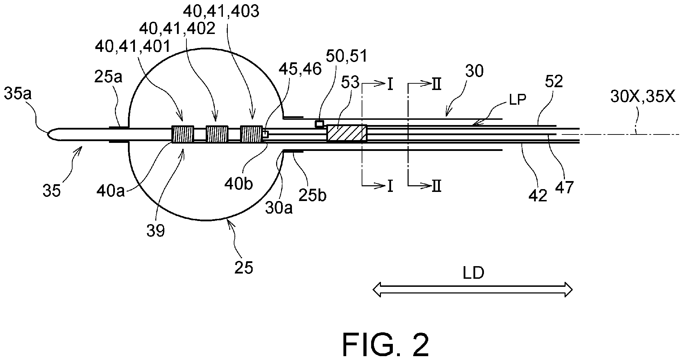

As shown in Fig. 2, the catheter body 20 in this embodiment has a balloon 25, a catheter shaft 28 to which the balloon 25 is attached, and a heating unit 39 located in the balloon 25. The catheter shaft 28 has an outer cylinder shaft 30 connected to the proximal end 25b of the balloon 25, and an inner cylinder shaft 35 connected to the distal end 25a of the balloon 25. The inner cylinder shaft 35 passes through the outer cylinder shaft 30 to extend into the balloon 25. A liquid flow path LP leading into the balloon 25 is formed between the outer cylinder shaft 30 and the inner cylinder shaft 35. The heating unit 39 heats the liquid in the balloon 25.

The longitudinal direction LD of the catheter body 20 is specified as the direction along which the center axes 30X, 35X of the outer cylinder shaft 30 and the inner cylinder shaft 35 extending from the outer cylinder shaft 30 extend. In this specification, the "distal" side used for respective structures of the ballon catheter 15 and the catheter body 20 means the side away from the operator (surgeon) operating the handle 60 and the balloon catheter 15 along the longitudinal direction LD of the catheter body 20, in other words, the distant end side. In addition, the "proximal" side used for respective structures of the balloon catheter 15 and the catheter body 20 means the side close to the operator (surgeon) operating the handle 60 and the balloon catheter 15 along the longitudinal direction LD of the catheter body 20, in order words, the near end side.

The balloon catheter system 10 and the balloon catheter 15 are described in more detail below. The catheter body 20 of the balloon catheter 15 is first described in detail. As described above, the catheter body 20 of the balloon catheter 15 according to this embodiment has the balloon 25, the outer cylinder shaft 30, the inner cylinder shaft 35 and the heating unit 39, as well as an electrode temperature sensor 45, and a liquid-flow-path temperature sensor 50.

The outer cylinder shaft 30 and the inner cylinder shaft 35 both have a cylindrical shape, typically, a circular cylindrical shape. Thus, the outer cylinder shaft 30 and the inner cylinder shaft 35 each form a lumen as an inner space. For example, a guide wire, not shown, is inserted through the lumen formed by the inner cylinder shaft 35. The inner cylinder shaft 35 is inserted through the lumen formed by the outer cylinder shaft 30. Namely, the outer cylinder shaft 30 and the inner cylinder shaft 35 have a double-tube shaft configuration. The internal diameter of the outer cylinder shaft 30 is larger than the external diameter of the inner cylinder shaft 35. Thus, a lumen remains between the outer cylinder shaft 30 and the inner cylinder shaft 35. The lumen between the outer cylinder shaft 30 and the inner cylinder shaft 35 forms the liquid flow path LP. As shown in Fig. 2, the liquid flow path LP is in communication with the inside of the balloon 25. In addition, the liquid flow path LP extends into the handle 60.

The lengths of the outer cylinder shaft 30 and the inner cylinder shaft 35 are each preferably 500 mm or more and 1700 mm or less, and more preferably, 600 mm or more and 1200 mm or less. The outer cylinder shaft 30 and the inner cylinder shaft 35 are preferably made of a flexible material with excellent anti-thrombogenic properties. Examples of the flexible material with excellent anti-thrombogenic properties include, but are not limited to, fluoropolymers, polyamides, polyurethane-based polymers, polyimides or the like. The outer cylinder shaft 30 is preferably made by laminating layers of different flexible materials, in order to ensure both sliding on the inner cylinder shaft 35 and adhesion or heat welding to the balloon 25.

The external diameter of the outer cylinder shaft 30 is preferably 3.0 mm or more and 4.0 mm or less. The internal diameter of the outer cylinder shaft 30 is preferably 2.5 mm or more and 3.5 mm or less. In addition, the external diameter of the inner cylinder shaft 35 is preferably 1.4 mm or more and 1.7 mm or less. The internal diameter of the inner cylinder shaft 35 is preferably 1.1 mm or more and 1.3 mm or less.

The balloon 25 is connected to the outer cylinder shaft 30 and the inner cylinder shaft 35. The balloon 25 is formed to inflate when it is filled with a liquid, and is formed to deflate when the liquid is discharged therefrom. The balloon 25 preferably has a shape to fit a target site to be treated (e.g., blood vessel). For example, the balloon 25 formed to be adapted to a pulmonary venous junction of a left atrium has a spherical shape having a diameter of 15 mm or more and 40 mm or less. The spherical shape includes a true spherical shape, an oblate spherical shape, and oblong spherical shape, and further a substantially spherical shape.

In a case where high-frequency current conduction electrodes 41 are employed as the heating electrodes 40 of the heating unit 39 as described below, a thinner film thickness of the balloon 25 is preferred in order to lower its impedance at a frequency of electric energy supplied by the control unit 70 serving as a high-frequency power supply means. However, if the film thickness of the balloon 25 is excessively thin, the balloon 25 may puncture when the surface of the balloon 25 is damaged . Thus, the film thickness of the balloon 25 is preferably 5 µm or more and 300 µm or less, and more preferably 10 µm or more and 200 µm or less. Note that the thickness of the balloon 25 is not limited to the above range, because the impedance of the balloon 25 varies greatly depending on the dielectric constant of the material forming the balloon 25 and the frequency of the aforementioned electric energy.

A material of the balloon 25 is preferably an elastic material with excellent anti-thrombogenic properties. Specifically, a polyurethane-based polymeric material or the like can be used. Examples of the polyurethane-based polymeric material applicable to the balloon 25 include thermoplastic polyether urethane, polyether polyurethane urea, fluorinated polyether urethane urea, polyether polyurethane urea resin, or polyether polyurethane urea amide.

In the illustrated catheter body 20, as shown in Figs. 2 to 4, the distal end (distant end) 25a of the balloon 25 is fixed on the distal end (distant end) 35a of the inner cylinder shaft 35. The proximal end (near end) 25b of the balloon 25 is fixed on the distal end (distant end) 30a of the outer cylinder shaft 30. The gap between the distal end 30a of the outer cylinder shaft 30 and the inner cylinder shaft 35 defines an opening that communicates the liquid flow path LP and the inner space of the balloon 25. The balloon 25 can be connected to the outside shaft 30 and the inside shaft 35 by adhesion or heat welding.

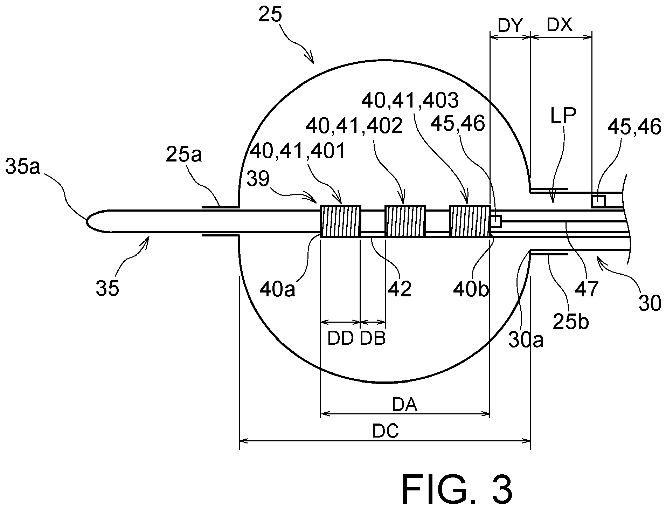

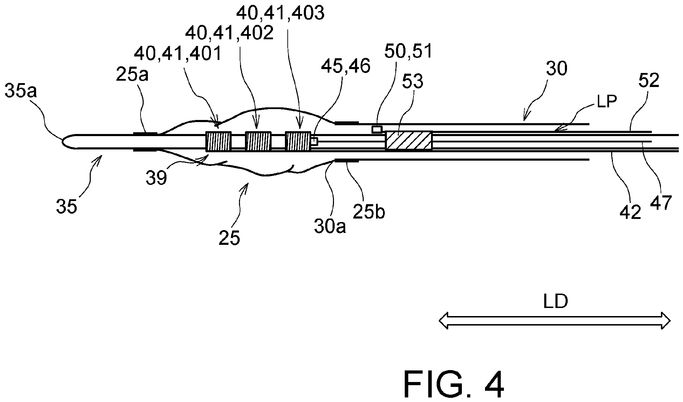

When the outer cylinder shaft 30 and the inner cylinder shaft 35 are relatively moved in relation to each other in the longitudinal direction LD, the balloon 25 connected to the outer cylinder shaft 30 and the inner cylinder shaft 35 is deformed. In the illustrated example, the size of the balloon 25 in the longitudinal direction LD can be adjusted by the relative movement between the outer cylinder shaft 30 and the inner cylinder shaft 35. As shown in Fig. 4, when the inner cylinder shaft 35 is relatively moved with respect to the outer cylinder shaft 30 toward the distal side in the longitudinal direction LD, the balloon 25 is stretched in the longitudinal direction LD to become taut. In the illustrated example, the distal movement range of the inner cylinder shaft 35 with respect to the outer cylinder shaft 30 in the longitudinal direction LD is restricted by the balloon 25. When the inner cylinder shaft 35 is relatively moved with respect to the outer cylinder shaft 30 from the position shown in Fig. 4 toward the proximal side in the longitudinal direction LD, the balloon 25 becomes loosened. By introducing a liquid into the loosened balloon 25, the balloon 25 can be inflated as shown in Figs. 2 and 3. Namely, the dimensions of the balloon 25 in the longitudinal direction LD can be adjusted by the relative movement between the outer cylinder shaft 30 and the inner cylinder shaft 35.

Next, the heating unit 39 is described. The heating unit 39 includes the heating electrodes 40. The heating electrodes 40 are located on the inner cylinder shaft 35 in the balloon 25. Each heating electrode 40 is a component for heating the liquid filled in the balloon 25. A nichrome wire that generates electrical resistance heat may be employed as the heating electrodes 40, for example. The high-frequency current conduction electrodes 41 may be employed as another example of the heating electrodes 40. Specifically, the high-frequency current conduction electrodes 41 are coil electrodes shown in Figs. 2 to 4. By performing the high-frequency current conduction (high-frequency energization) to the high-frequency current conduction electrodes 41 as the heating electrodes 40, a high-frequency current flows between these electrodes 41 and a counter electrode (return pad) 77 (see Fig. 1) disposed outside, so that the liquid positioned between the high-frequency current conduction electrodes 41 and the counter electrode 77 generates Joule heat. The counter electrode 77 is located on the back of a patient, for example.

In the example shown in Figs. 2 to 4, the high-frequency current conduction electrodes 41 serving as the heating electrodes 40 are electrically connected to a lead wire 42 which is a high-frequency current conduction lead wire for high-frequency current conduction. The lead wire 42 extends in the liquid flow path LP, which is the lumen between the outer cylinder shaft 30 and the inner cylinder shaft 35, up to the handle 60.

In the example shown in Figs. 2 to 4, the high-frequency current conduction electrodes 41 are provided on the outer circumferential surface of the inner cylinder shaft 35 extending inside the balloon 25. The high-frequency current conduction electrodes 41, each of which is a coil electrode, may be formed by a conducting wire wound around the inner cylinder shaft 35. The high-frequency current conduction electrodes 41 are electrically connected to the lead wire 42 for high-frequency current conduction. A specific example of coil electrodes forming the high-frequency current conduction electrodes 41 may be coil electrodes that are made as follows. An insulation-coated lead wire used as the lead wire 42 is stripped of its coating. Then, the lead wire is wound around the inner cylinder shaft 35 to provide coil electrodes. Since such the high-frequency current conduction electrodes 41 are integrally formed with the lead wire 42, occurrence of trouble such as disconnection or open circuit of wire can be effectively avoided.

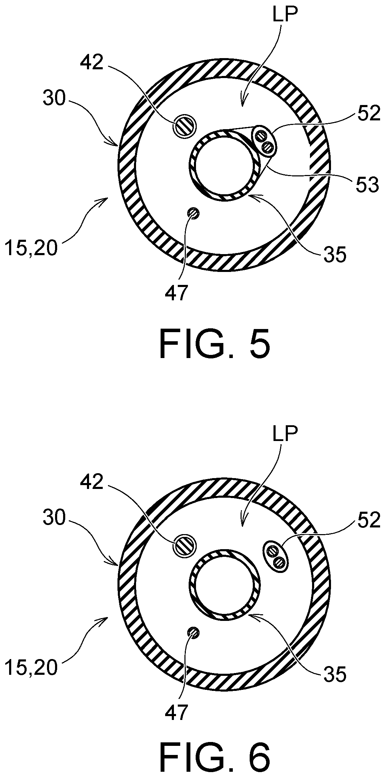

Diameters of the high-frequency current conduction electrodes 41 as coil electrodes and the lead wire 42 are preferably between 0.1 mm or more and 1 mm or less, and more preferably between 0.1 mm or more and 0.4 mm or less. A conductive material forming the high-frequency current conduction electrodes 41 and the lead wire 42 may be, for example, copper, silver, gold, platinum, and alloys thereof. In order to prevent a short circuit, a conductive linear portion of the lead wire 42 is preferably covered with an insulating coat made of fluoropolymer, for example (see Figs. 5 and 6).

Next, the electrode temperature sensor 45 is described. The electrode temperature sensor 45 acquires information on the temperature of the heating electrodes 40. In the illustrated example, the electrode temperature sensor 45 has a thermosensitive part 46 attached to the proximal end 40a of the heating electrodes 40. The information acquired by the electrode temperature sensor 45 is used for controlling the high-frequency current conduction (supply of electric energy) to the heating electrodes 40, which is important in an ablation therapy using the balloon catheter system 10.

A thermocouple or thermistor can be employed as the electrode temperature sensor 45. In particular, the electrode temperature sensor 45 is preferably a T-type thermocouple, which allows reduction in heat capacity of its thermosensitive part 46. In addition, when a T-type thermocouple is employed as the electrode temperature sensor 45, it stabilizes a thermal electromotive force. Further, since a T-type thermocouple can highly precisely detect the temperature range between 50°C and 80°C, it is particularly suitable for a cardiac ablation therapy. Information on a temperature acquired by the electrode temperature sensor 45 is, for example, an electric potential that can be acquired from a thermocouple, or a resistance value that can be acquired from a thermistor.

As shown in Figs. 2 to 4, the electrode temperature sensor 45 typically has the thermosensitive part 46, and a lead wire 47 electrically connected to the thermosensitive part 46. When the electrode temperature sensor 45 comprises a thermocouple, the part where different metals are connected serves as the thermosensitive part 46. When the electrode temperature sensor 45 comprises a thermistor, its ceramic element serves as the thermosensitive part 46. The lead wire 47 extends in the liquid flow path LP, which is the lumen between the outer cylinder shaft 30 and the inner cylinder shaft 35, up to the handle 60.

The diameter of the lead wire 47 is preferably 0.05 mm or more and 0.5 mm or less, and more preferably 0.05 mm or more and 0.3 mm or less. The electrode temperature sensor 45 as a thermocouple is formed by joining the high-frequency current conduction electrode 41 and the lead wire 47. In this case, the high-frequency current conduction electrode 41 may be of copper and the lead wire 47 may be of constantan. In this example, the thermosensitive part 46 where the high-frequency current conduction electrode 41 and the lead wire 47 are joined can function as a T-type thermocouple. In order to prevent a short circuit with other lead wires 42, 52 located in the liquid flow path LP, they are preferably provided with an electrically insulating coat made of fluoropolymer, enamel, etc., as shown in Figs. 5 and 6.

Next, the liquid-flow-path temperature sensor 50 is described. The liquid-flow-path temperature sensor 50 acquires information on a temperature of the liquid. In this embodiment, the liquid flow path temperature 50 has a thermosensitive part 51 located in the liquid flow path LP between the outer cylinder shaft 30 and the inner cylinder shaft 35. The liquid-flow-path temperature sensor 50 can acquire information on the temperature of the liquid in the liquid flow path LP. The information acquired by the liquid-flow-path temperature sensor 50 is used for estimating the surface temperature of the balloon 25, which is important in the ablation therapy using the balloon catheter system 10.

As described later, in the illustrated example, the surface temperature of the balloon 25 is determined based on the temperature of the liquid discharged from the balloon 25 into the liquid flow path LP. For the purpose of accurately determining the surface temperature of the balloon 25, it is preferable to determine, in a strict sense, the distance DX (see Fig. 3) from the distal end 30a of the outer cylinder shaft 30 up to the thermosensitive part 46 of the liquid-flow-path temperature sensor 50 along the longitudinal direction LD based on the amount of the liquid supplied and discharged by the agitator 75 described later. In more detail, within the liquid flow path LP between the outer cylinder shaft 30 and the inner cylinder shaft 35, the thermosensitive part 51 is preferably positioned in the area into which the liquid in the balloon 25 is drawn when the liquid is suctioned by the agitator 75 (the area at which the liquid discharged from the balloon 25 arrives). This enables the temperature of the liquid discharged from the balloon 25 to be detected by the liquid-flow-path temperature sensor 50 in the outer cylinder shaft 30. Specifically, the distance DX from the distal end 30a of the outer cylinder shaft 30 up to the liquid-flow-path temperature sensor 50 preferably is not more than the value obtained by dividing the amount of the liquid [mm³] discharged by the agitator 75 from the liquid flow path LP (liquid discharge amount) by a cross-sectional area [mm²] of the liquid flow path LP. Note that, in consideration of the respective dimensions of the balloon catheter 15 generally applied to the cardiac ablation therapy and the amount of the liquid supplied and discharged by the agitator 75, the distance DX from the distal end 30a of the outer cylinder shaft 30 up to the liquid-flow-path temperature sensor 50 is preferably 5 mm or more and 150 mm or less, and more preferably 10 mm or more and 20 mm or less.

Unless otherwise described, the distance DX from the distal end 30a of the outer cylinder shaft 30 up to the liquid-flow-path temperature sensor 50 is the length that is measured with respect to the balloon 25 inflated with a liquid as shown in Figs. 2 and 3. Similarly, unless otherwise described, it is determined based on the balloon 25 inflated with a liquid as shown in Figs. 2 and 3 whether the liquid-flow-path temperature sensor 50 is positioned in the liquid flow path LP, whether the liquid-flow-path temperature sensor 50 is positioned in the outer cylinder shaft 30 and whether the liquid-flow-path temperature sensor 50 is positioned between the outer cylinder shaft 30 and the inner cylinder shaft 35.

A thermocouple or thermistor can be employed as the liquid-flow-path temperature sensor 50. In particular, the liquid-flow-path temperature sensor 50 is preferably a T-type thermocouple, which allows reduction in heat capacity of the thermosensitive part 51. In addition, when a T-type thermocouple is employed as the liquid-flow-path temperature sensor 50, it stabilizes a thermal electromotive force. Further, since a T-type thermocouple can highly precisely detect the temperature range between 50°C and 80°C, it is particularly suitable for a cardiac ablation therapy. Information on the temperature acquired by the liquid-flow-path temperature sensor 50 is, for example, an electric potential that can be acquired from a thermocouple, or a resistance value that can be acquired from a thermistor.

As shown in Figs. 2 to 4, the liquid-flow-path temperature sensor 50 typically has the thermosensitive part 51, and lead wire(s) 52 electrically connected to the thermosensitive part 51. When the liquid-flow-path temperature sensor 50 comprises a thermistor, the part to which different metals are connected serves as the thermosensitive part 51. When the liquid-flow-path temperature sensor 50 comprises a thermistor, its ceramic element serves as the thermosensitive part 51. The lead wire(s) 51 extend in the liquid flow path LP, which is the lumen between the outer cylinder shaft 30 and the inner cylinder shaft 35, up to the handle 60.

The diameter of the lead wire(s) 52 is preferably 0.05 mm or more and 0.5 mm or less, and more preferably 0.05 mm or more and 0.3 mm or less. In the liquid-flow-path temperature sensor 50 as a thermocouple, one lead wire 52 may be of copper and the other lead wire 52 may be of constantan. In this example, the thermosensitive part 51 formed by joining a pair of the lead wires 52 can function as a T-type thermocouple. In order to prevent a short circuit between the lead wires 52, or in order to prevent a short circuit between the lead wire(s) 52 and other lead wires 42, 47, they are preferably provided with an electrically insulating coat made of fluoropolymer, enamel, etc., as shown in Figs. 5 and 6. The thermosensitive part 51 of the liquid-flow-path temperature sensor 50 may be formed by joining a lead wire 52 and the lead wire 42.

In the illustrated example, the liquid-flow-path temperature sensor 50 is attached to the inner cylinder shaft 35. As shown in Figs. 2 to 4, the lead wire(s) 52 of the liquid-flow-path temperature sensor 50 is fixed so that the liquid-flow-path temperature sensor 50 is attached to the inner cylinder shaft 35. Note that the thermosensitive part 51 is separated from both the outer cylinder shaft 30 and the inner cylinder shaft 35. In other words, the thermosensitive part 51 is not in contact with either of the outer cylinder shaft 30 or the inner cylinder shaft 35. This avoids the possibility that the thermosensitive part 51 detects the temperature of the outer cylinder shaft 30 or the inner cylinder shaft 35, which have a large heat capacity, thereby worsening responsiveness of the liquid-flow-path temperature sensor 50. As a result, it is possible to ensure high responsiveness of the liquid-flow-path temperature sensor 50 to the temperature of the liquid in the liquid flow path LP. A fixing means 53 for fixing the lead wire(s) 52 onto the inner cylinder shaft 35 is not particularly limited, and various means can be used. In the illustrated example, a heat-shrinkable tube that shrinks by heat is used as the fixing means 35. However, not limited to this example, various types of shrinkable tube, an adhesive tape, a glue, etc. can be used as the fixing means 53.

In the example shown in Figs. 2 to 4, in a state where the inner cylinder shaft 35 is relatively moved with respect to the outer cylinder shaft 30 toward the distal side at a maximum in the longitudinal direction in order that the balloon 25 is stretched, the liquid-flow-path temperature sensor 50 is positioned in the outer cylinder shaft 30. This specific example allows the liquid-flow-path temperature sensor 50 to be positioned in the outer cylinder shaft 30 irrespective of the relative positions of the inner cylinder shaft 35 with respect to the outer cylinder shaft 30. Thus, the liquid-flow-path temperature sensor 50 can be stably protected by the outer cylinder shaft 30 irrespective of the relative positions of the inner cylinder shaft 35 with respect to the outer cylinder shaft 30.

Differently from the illustrated example, the liquid-flow-path temperature sensor 50 may be attached to the outer cylinder shaft 30. For example, the lead wire(s) 52 of the liquid-flow-path temperature sensor 50 may be fixed on the inner surface of the outer cylinder shaft 30. In this case, the liquid-flow-path temperature sensor 50 is positioned in the outer cylinder shaft 30 irrespective of the relative poisons of the inner cylinder shaft 35 with respect to the outer cylinder shaft 30. Thus, the liquid-flow-path temperature sensor 50 can be stably protected by the outer cylinder shaft 30 irrespective of the relative positions of the inner cylinder shaft 35 with respect to the outer cylinder shaft 30.

Next, the handle 60, which is connected from the proximal side to the aforementioned catheter body 20, is described. The handle 60 is the part held by an operator (surgeon) during the use of the balloon catheter system 10. Thus, the handle 60 preferably has a design that allows an operator to easily grasp and operate the handle 60 in his/her hand(s). The handle 60 is preferably made by a material of excellent chemical resistance, such as polycarbonate and ABS resin.

The handle 60 shown in Fig. 1 has a first handle part 61 and a second handle part 62 that are slidable to each other. The first handle part (front handle part) 61 is connected to the outer cylinder shaft 30 of the catheter body 20. The second handle part (rear handle part) 62 is connected to the inner cylinder shaft 35 of the catheter body 20. By relatively moving the second handle part 62 with respect to the first handle part 61, the inner cylinder shaft 35 can be relatively moved with respect to the outer cylinder shaft 30.

As shown in Fig. 1, the handle 60 also functions as a part that connects the balloon catheter 15 and the other devices included in the balloon catheter system 10.

A connector 66 extends from the second handle part 62. This connector 66 electrically connects the lead wire 42 of the heating electrodes 40, the lead wire 47 of the electrode temperature sensor 45, and the lead wire(s) 52 of the liquid-flow-path temperature sensor 50 to the control unit 70 described later.

As shown in Fig. 1, an extension tube 67 extends from the first handle part 61. The extension tube 67 communicates the liquid flow path LP of the catheter body 20 with the supply unit 74 or the agitator 75 described later. The extension tube 67 is connected to the supply unit 74 and the agitator 75 via a valve 68. In the illustrated example, whether the supply unit 74 or the agitator 75 is communicated with the liquid flow path LP can be selected by operating the valve 68. A three-way stopcock may be used as the valve 68.

Next, the devices constituting the balloon catheter system 10 together with the aforementioned balloon catheter 15, specifically, the control unit 70, the supply unit 74, and the agitator 75, are described.

The illustrated control unit 70 is electrically connected to the lead wire 42 of the heating electrodes 40 and the lead wire 47 of the electrode temperature sensor 45. The control unit 70 has a high-frequency current conduction controller 70a that controls high-frequency current conduction to the high-frequency current conduction electrodes 41. In the illustrated example, the high-frequency current conduction controller 70a controls the high-frequency current conduction (supply of electric energy) to the high-frequency current conduction electrodes 41 in order to adjust an output from the heating electrodes 40. The high-frequency current conduction controller 70a can control the high-frequency current conduction to the high-frequency current conduction electrodes 41, based on information on the temperature of the heating electrodes 40 acquired from the electrode temperature sensor 45, or based on the surface temperature of the balloon 25 which is estimated by a below-described temperature calculation unit 70b (referred to also as "estimated surface temperature" hereunder), or in accordance with a preset process, or in accordance with an input from an operator. In the illustrated example, the high-frequency current conduction controller 70a controls the high-frequency current conduction to the heating electrodes 40 based on the temperature of the heating electrodes 40 which is set by an operator (referred to also as "set electrode temperature") and the temperature of the heating electrodes 40 which is detected by the electrode temperature sensor 45. In addition, in the illustrated example, the high-frequency current conduction controller 70a automatically controls the amount of electric energy supplied to the heating electrodes 40, such that, when the temperature of the heating electrodes 40 detected by the electrode temperature sensor 45 becomes equal to or greater than a predetermined value, the amount of electric energy supplied to the heating electrodes 40 decreases, and that, when the temperature of the heating electrodes 40 detected by the electrode temperature sensor 45 falls below the predetermined value, the amount of electric energy supplied to the heating electrodes 40 increases.

In addition, the control unit 70 is electrically connected to the lead wire(s) 52 of the liquid-flow-path temperature sensor 50. The control unit 70 has the temperature calculation unit 70b that performs calculation based on the temperature information acquired by the liquid-flow-path temperature sensor 50. The temperature calculation unit 70b calculates the temperature of the liquid in the liquid flow path LP based on the temperature information acquired by the liquid-flow-path temperature sensor 50, and further estimates the estimated surface temperature of the balloon 25 based on the calculated liquid temperature. The temperature calculation unit 70b may display the estimated surface temperature of the balloon 25 on a display 71.

The control unit 70 further has an agitator controller 70c that controls the agitator 75. The agitator controller 70c may display a control condition of the agitator 75 on the display 71.

The control unit 70 is constituted by hardware such as a CPU, for example. One or more of the high-frequency current conduction controller 70a, the temperature calculation unit 70b and the agitator controller 70c, which are included in the control unit 70, may be constituted by separate hardware or may share a part. At least a part of the control unit 70 may be constituted by software. A part of the control unit 70 may be physically separated from the remaining. A component of the control unit 70 may be able to cooperate with another component of the control unit 70 by communication through a network. A component of the control unit 70 may be on a device, such as a server or database on a cloud platform, which can communicate with another component of the control unit 70 through an external network.

Next, the supply unit 74 is described. The supply unit 74 supplies a liquid into the liquid flow path LP. By supplying a liquid from the supply unit 74 to the balloon 25 through the liquid flow path LP, the balloon 25 can be inflated as shown in Figs. 2 and 3. On the other hand, by discharging a liquid from the balloon 25 to the supply unit 74 through the liquid flow path LP, the balloon 25 can be deflated. The liquid to be supplied into the liquid flow path LP may typically be a physiological saline solution. The liquid to be supplied into the liquid flow path LP may be a contrast agent diluted with saline so that the balloon 25 inflated with the liquid can be seen on a radiographic image. A syringe can be used as the supply unit 74 as illustrated. However, a pump or the like can also be used as the supply unit 74.

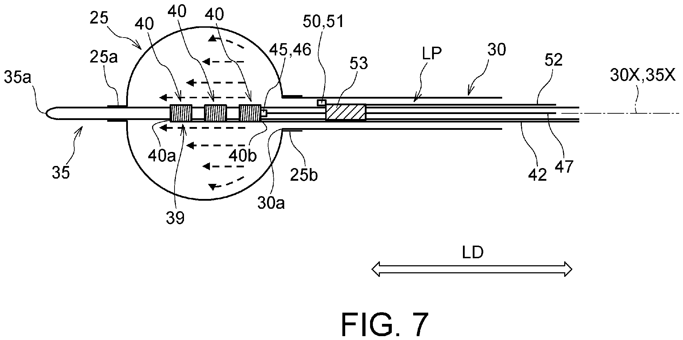

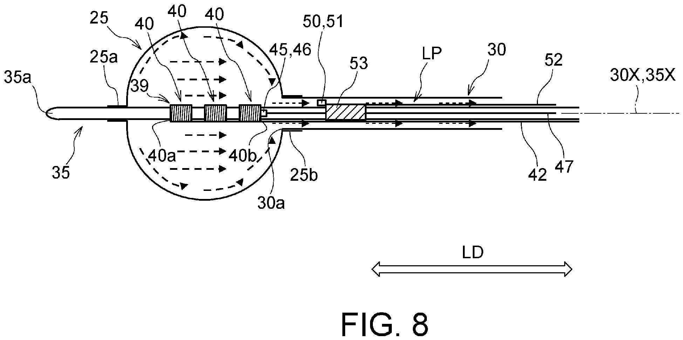

Next, the agitator 75 is described. The agitator 75 is provided for agitating the liquid in the balloon 25. By agitating the liquid in the balloon 25, the heated liquid around the heating electrodes 40 is diffused so that the surface temperature of the balloon 25 can be adjusted. The agitator 75 repeats supply of a liquid to the liquid flow path LP and discharge of a liquid from the liquid flow path LP. When a liquid is supplied from the agitator 75 to the liquid flow path LP, it causes the liquid flow path LP to supply a liquid into the balloon 25 (see Fig. 7). When a liquid is discharged from the liquid flow path LP to the agitator 75, it causes the balloon 25 to discharge a liquid to the liquid flow path LP (see Fig. 8). The liquid in the balloon 25 is agitated by repeating the supply of a liquid to the balloon 25 and the discharge of a liquid from the balloon 25. The agitator 75 can be a pump selected from the group consisting of a roller pump, a diaphragm pump, a bellows pump, a vane pump, a centrifugal pump, and a pump comprising a piston and a cylinder in combination. In Figs. 7 and 8, the dotted arrows show flow of the liquid.

The amount of a liquid to be supplied to the liquid flow path LP and the amount of a liquid to be discharged from the liquid flow path LP may be a predetermined amount (e.g., between 5 ml or more and 30 ml or less). Supply of a liquid to the liquid flow path LP and discharge of a liquid from the liquid flow path LP may be repeated at a regular cycle (e.g., between once or more and five times or less per second). The amount of a liquid to be supplied to the liquid flow path LP and the amount of a liquid to be discharged from the liquid flow path LP may be adjusted by a control signal from the aforementioned agitator controller 70c or by a direct input made by an operator. Similarly, the time length of a cycle at which a liquid is supplied to the liquid flow path LP and a liquid is discharged from the liquid flow path LP may be adjusted by a control signal from the aforementioned agitator controller 70c or by an input directly made by an operator.

Next, an example of the use of the balloon catheter system 10 as structured above is described.

The valve 68 is first operated such that the supply unit 74 communicates with the liquid flow path LP of the catheter body 20 through the handle 60 and the extension tube 67. Then, the supply unit 74 is operated to allow a liquid to flow into the liquid flow path LP, so that the balloon 25, the liquid flow path LP, and the extension tube 67 are each filled with a liquid. Thereafter, the inner cylinder shaft 35 is relatively moved with respect to the outer cylinder shaft 30 to the distal side (distant side) in the longitudinal direction LD, so that the balloon 25 is stretched as shown in Fig. 4. At this time, by operating the first handle part 61 and the second handle part 62 of the handle 60, the outer cylinder shaft 30 and the inner cylinder shaft 35 can be relatively moved with respect to each other. Then, the catheter body 20 with the stretched balloon 25 is inserted into a patient's body.

When the distal end of the catheter body 20 has been guided close to a target site (diseased area), the inner cylinder shaft 35 is relatively moved with respect to the outer cylinder shaft 30 to the proximal side (near side) in the longitudinal direction LD, so that the balloon 25 is loosened. Then, the valve 68 is operated to communicate the supply unit 74 with the liquid flow path LP of the catheter body 20 through the handle 60. Thereafter, the supply unit 74 is operated to allow a liquid to flow into the liquid flow path LP, so that the balloon 25 is inflated with a liquid as shown in Figs. 2 and 3.

Then, the valve 68 is operated to shut off the supply unit 74 from the liquid flow path LP, and to communicate the agitator 75 with the liquid flow path LP. The agitator 75 is controlled by a control signal from the agitator controller 70c of the control unit 70. The agitator 75 repeats supply of a predetermined amount of a liquid to the liquid flow path LP and discharge of a predetermined amount of a liquid from the liquid flow path LP at a regular cycle. This results in repeated injection of the predetermined amount of a liquid from the liquid flow path LP into the balloon 25 and suction of the predetermined amount of a liquid from inside the balloon 25 to the liquid flow path LP at the regular cycle. Thus, the liquid in the balloon 25 is agitated.

In addition, the liquid temperature in the balloon 25 is adjusted by controlling the heating component 40 by means of the high-frequency current conduction controller 70a of the control unit 70. Specifically, a high-frequency current is conducted from the control unit 70 between the high-frequency current conduction electrodes 41 forming the heating electrodes 40 and the counter electrode 77 located outside the body of a patient. As a result, a high-frequency current is generated between the high-frequency current conduction electrode 41 and the counter electrode 77. Since the size of the high-frequency current conduction electrodes 41 are significantly smaller than that of the current electrode 77, a current density around the high-frequency current conduction electrodes 41 increases, and the liquid around the high-frequency current conduction electrodes 41 is heated by Joule heat.

The liquid in the balloon 25 is heated and agitated as described above. Then, the balloon 25 containing the heated liquid is brought into contact with the target site to ablate the target site. During the ablation, the electrode temperature sensor 45 attached to the heating electrodes 40 acquires information on the temperature of the heating electrodes 40. Supply of electric energy to the heating electrodes 40 is controlled based on the information acquired by the electrode temperature sensor 45. In addition, during the ablation, the liquid-flow-path temperature sensor 50 located in the liquid flow path LP acquires information on the liquid temperature in the liquid flow path LP. The temperature calculation unit 70b of the control unit 70 performs calculation based on the acquired information to derive the estimated surface temperature of the balloon 25. The estimated surface temperature of the balloon 25 obtained by the temperature calculation unit 70b is displayed on the display 71, for example.

Upon completion of the ablation to the target site, the supply of electric energy to the heating electrodes 40 is stopped. In addition, the valve 68 is operated so that the supply unit 74 is communicated with the liquid flow path LP of the catheter body 20 through the handle 60, and that the agitator 75 is shut off from the liquid flow path LP. Then, the liquid is discharged by means of the supply unit 74 from the liquid flow path LP to deflate the balloon 25. Then, the second handle part 62 is operated to stretch the deflated balloon 25 as shown in Fig. 4. After that, the catheter body 20 with the stretched balloon 25 is pulled out from the patient's body. In this manner, the procedure using the balloon catheter system 10 is completed.

Since it is possible to control the surface temperature of the balloon 25 reliably, it is also possible to adjust the surface temperature of the balloon 25 to an ideal temperature during the procedure, which enables the ablation therapy to become significantly effective. However, it sometimes actually occurs that the surface temperature of the balloon 25 cannot be increased as desired, although the supply of electric energy to the heating electrodes 40 is controlled by the high-frequency current conduction controller 70a of the control unit 70.

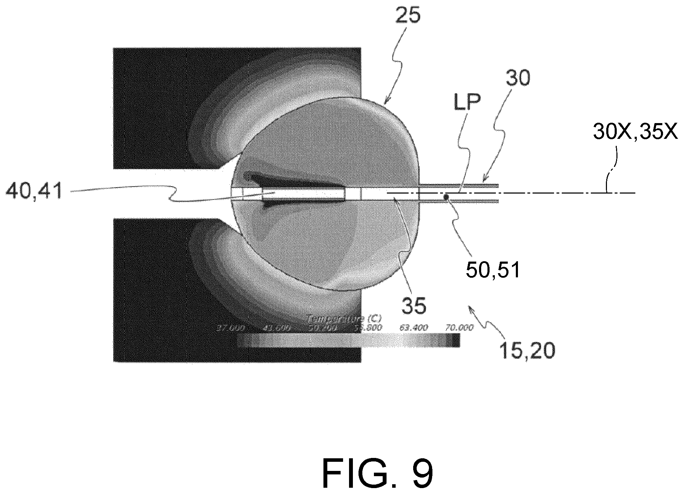

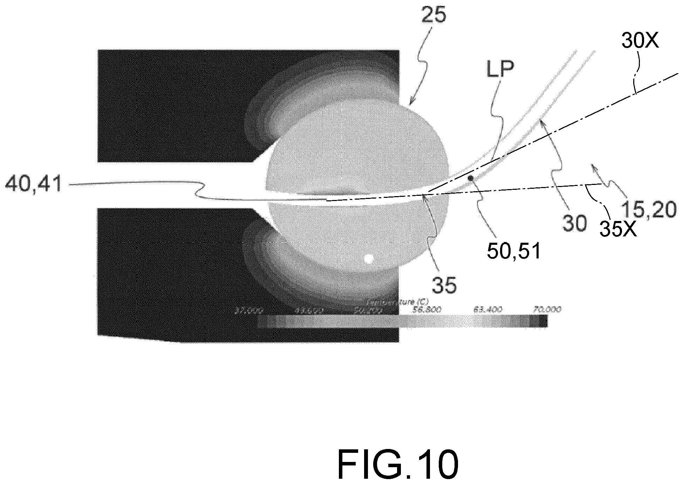

The following was found by intensive study of the inventors. Namely, when the balloon 25 is pressed against the target site during the ablation, the angle of the center axis 30X of the outer cylinder shaft 30 at the distal end 30a of the outer cylinder shaft 30, with respect to the center axis 35X of the inner cylinder shaft 35 in the balloon 25, varies depending on the angle at which the balloon 25 is pressed against the target site (pressing angle). For example, as shown in Fig. 9, the ballon 25 may be pressed against the target site in a state where the center axis 35X and the center axis 30X are coincident to each other (this state is referred to also as "coaxial state" hereunder). On the other hand, as shown in Fig. 10, the balloon 25 may also be pressed against the target site in a state where the center axis 30X is inclined to the center axis 35X (this state is referred to also as "non-coaxial state" hereunder). In a conventional balloon catheter, the surface temperature of the balloon 25 may differ significantly between a case in which the balloon 25 is pressed against the target site in the coaxial state and a case in which the balloon 25 is pressed against the target site in the non-coaxial state, even when a set electrode temperature of the heating electrode 40 set by the high-frequency current conduction controller 70a is the same. Specifically, when the balloon 25 is pressed against the target site in the non-coaxial state, the surface temperature of the balloon 25 may be significantly lower than the case in which the balloon 25 is pressed against the target site in the coaxial state.

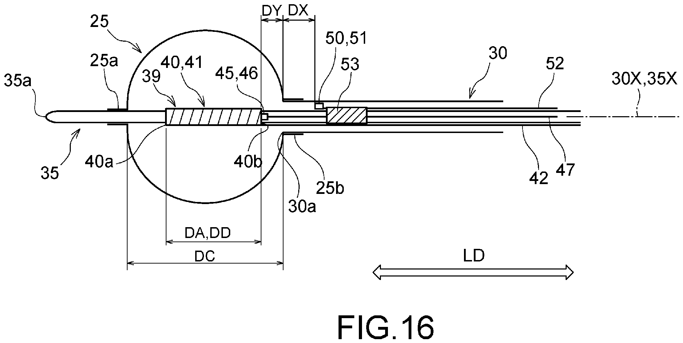

The reason thereof is considered as follows. Namely, also in the case of the conventional balloon catheter shown in Fig. 16, when the balloon 25 is pressed against the target site in the coaxial state, orientation of the distal end 30a of the outer cylinder shaft 30 follows the direction in which the inner cylinder shaft 35 is extended in the balloon 25. Thus, as shown in Fig. 7, when a liquid is supplied from the agitator 75 to the liquid flow path LP, a liquid supplied from the liquid flow path LP to the balloon 25 flows in the balloon 25 along the heating electrode 40 on the inner cylinder shaft 35 to diffuse the heated liquid around the heating electrode 40. As a result, the surface temperature of the balloon 25 increases. In addition, since the liquid supplied from the liquid flow path LP to the balloon 25 flows along the heating electrode 40, the electrode temperature sensor 45 attached to the heating electrode 40 is efficiently cooled by the aforementioned liquid. As a result, the supply of electric energy from the control unit 70 to the heating electrode 40 is continued.

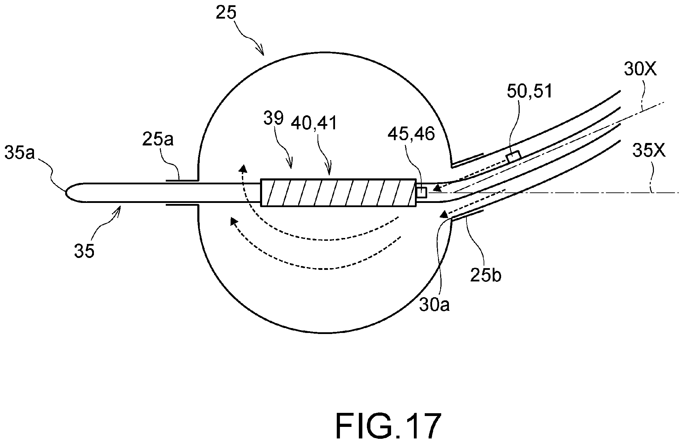

However, in the case of the conventional balloon catheter shown in Fig. 16, when the balloon 25 is pressed against the target site in the non-coaxial state, as shown in Fig. 17, the orientation of the distal end 30a of the outer cylinder shaft 30 deviates from the direction in which the inner cylinder shaft 35 is extended in the balloon 25. Thus, when a liquid is supplied from the agitator 75 to the liquid flow path LP, the flow of a liquid supplied from the liquid flow path LP to the balloon 25 deviates from the heating electrode 40 on the inner cylinder shaft 35, so that the heated liquid around the heating electrode 40 cannot be diffused, or the aforementioned heated liquid cannot be efficiently diffused. As a result, the surface temperature of the balloon 25 does not increase as desired. In addition, the electrode temperature sensor 45 attached to the heating electrode 40 on the inner cylinder shaft 35 is not cooled by the liquid supplied from the liquid flow path LP to the balloon 25. As a result, the amount of electric energy supplied from the control unit 70 to the heating electrode 40 decreases, so that the liquid heating effect by the heating electrode 40 and the counter electrode 77 decreases. In Fig. 17, illustration of the lead wires 42, 47, 52 is omitted for the sake of clarification. In Fig. 17, the dotted arrows show the flow of the liquid.

In consideration of these circumstances, the balloon catheter 15 in this embodiment is designed as follows, in order to prevent the surface temperature of the balloon 25 from greatly varying depending on the angles at which the balloon 25 is pressed against a target site during ablation.

Conventionally, as shown in Fig. 16, a single long heating electrode 40 serving as the heating unit 39 is located on the inner cylinder shaft 35. On the other hand, in the balloon catheter 15 in this embodiment, as shown in Figs. 2 and 3, the heating unit 39 includes multiple short heating electrodes 401, 402, 403. In the balloon 25, the multiple heating electrodes 401, 402, 403 are distributed over the outer circumferential surface of the inner cylinder shaft 35 along the direction in which the inner cylinder shaft 35 extends. In other words, in the balloon 25, the multiple heating electrodes 401, 402, 403 are located away from each other on the inner cylinder shaft 35. The total length of the multiple heating electrodes 401, 402, 403 (sum of the lengths DD of each of the heating electrodes 401, 402, 403) is substantially the same as the length DD of the conventional single heating electrode 40 shown in Fig. 16. In addition, the electrode temperature sensor 45 is attached to the proximal end 40b of the multiple heating electrodes 401, 402, 403. In this specification, "the proximal end 40b of the multiple heating electrodes 401, 402, 403" is a proximal end of the most proximally located heating electrode 403 of the multiple heating electrodes 401, 402, 403.

Since the multiple heating electrodes 40 are distributed over the inner cylinder shaft 35, flexibility of the inner cylinder shaft 35 in the balloon 25 improves as compared with the case shown in Figs. 16 and 17. Namely, in the case shown in Fig. 16, the long heating electrode 40 is located on the inner cylinder shaft 35. The inner cylinder shaft 35 cannot be easily bent at the position where the heating electrode 40 is located. Thus, as shown in Fig. 17, the flexibility of the inner cylinder shaft 35 in the balloon 25 is low. On the other hand, in this embodiment, the multiple short heating electrodes 401, 402, 403 are distributed over the inner cylinder shaft 35. In this case, the inner cylinder shaft 35 can be easily bent at the positions between the heating electrodes 401, 402, 403. Thus, the flexibility of the inner cylinder shaft 35 in the balloon 25 improves.

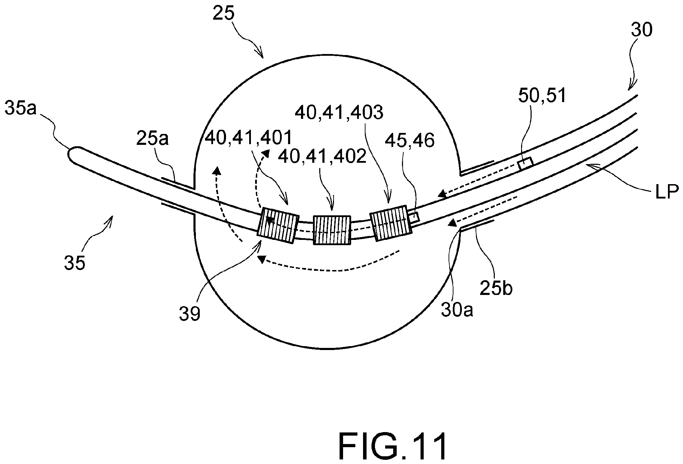

In this embodiment, due to the improvement in flexibility of the inner cylinder shaft 35 in the balloon 25, when the balloon 25 is pressed against a target site in the non-coaxially state, the inner cylinder shaft 35 can be smoothly curved in the balloon 25 from the distal end 25a to the proximal end 25b of the balloon 25. In this case, the inner cylinder shaft 35 is curved in accordance with the angle at which the balloon 25 is pressed against the target site. The distal end 30a of the outer cylinder shaft 30 is also orientated in accordance with the aforementioned pressing angle. Thus, the inner cylinder shaft 35 is curved along the flow of the liquid supplied from the liquid flow path LP into the balloon 25 through the distal end 30a of the outer cylinder shaft 30. As a result, as shown in Fig. 11, the flow of the liquid supplied from the liquid flow path LP to the balloon 25 follows the heating electrodes 40 on the inner cylinder shaft 35, as compared with the case shown in Figs. 16 and 17, so that the heated liquid around the heating electrodes 40 can be diffused. Thus, the surface temperature of the balloon 25 can be increased. In addition, since the liquid supplied from the liquid flow path LP to the balloon 25 flows through the heating electrodes 40, the heating electrodes 40 and the electrode temperature sensor 45 attached thereto are cooled by the aforementioned liquid. As a result, the amount of electric energy supplied from the control unit 70 to the heating electrodes 40 is maintained, so that the liquid heating effect (Joule heat generated in the liquid) by the heating electrodes 40 and the counter electrode 77 is not decreased. In Fig. 11, illustration of the lead wires 42, 47, 52 is omitted for the sake of clarification. In Fig. 11, the dotted arrows shown the flow of the liquid.

In addition, since the multiple heating electrodes 401, 402, 403 are distributed over the inner cylinder shaft 35, the length DA of the area in which the heating electrodes 40 are located on the inner cylinder shaft 35 can be elongated as compared with the case shown in Figs. 16 and 17. Thus, the liquid in the balloon 25 can be more uniformly heated along the longitudinal direction LD than the case shown in Figs. 16 and 17. Further, the proximal end 40b of the heating electrodes 40 can be brought closer to the distal end 30a of the outer cylinder shaft 30 than the case shown in Figs. 16 and 17. In other words, the distance between the proximal end 40b of the heating electrodes 40 and the distal end 30a of the outer cylinder shaft 30 (i.e., the distance between the electrode temperature sensor 45 and the distal end 30a of the outer cylinder shaft 30) DY can be shortened. As a result, the most proximally located heating electrode 403 and the electrode temperature sensor 45 attached thereto can be efficiently cooled by the liquid supplied from the distal end 30a of the outer cylinder shaft 30 into the balloon 25, irrespective of the angles at which the balloon 25 is pressed against the target site. In this specification, "the length of the area in which the heating electrodes 40 is located" is the distance between the distal end of the most distally located heating electrode 401 of the multiple heating electrodes 401, 402, 403 located on the inner cylinder shaft 35, and the proximal end of the most proximally located heating electrode 403. On the other hand, in the case shown in Figs. 16 and 17, "the length of the area in which the heating electrode 40 is located" is equal to the length DD of the heating electrode 40.

In the illustrated example, the multiple heating electrodes 40 are electrically connected to the single lead wire 42. In other words, electric energy is supplied to the multiple heating electrodes 40 through the common lead wire 42. This can avoid locating of multiple lead wires 42 in the liquid flow path LP. When multiple lead wires 42 are located in the liquid flow path LP, they narrow the space in the liquid flow path LP through which the liquid flows. Otherwise, when multiple lead wires 42 are located in the liquid flow path LP, the outer cylinder shaft 30 must be widened in order to secure the space in the liquid flow path LP through which the liquid flows.

In the illustrated example, each heating electrode 401, 402, 403 is a coil electrode. The multiple coil electrodes as the multiple heating electrodes 401, 402, 403 are formed by winding the single lead wire 42 around the inner cylinder shaft 35 at multiple locations thereon. This produces the multiple heating electrodes 401, 402, 403 which are electrically connected by the single lead wire 42.

A spacer (not shown) may be located between the adjacent heating electrodes 401, 402; 402, 403, in order to maintain the distance between the adjacent heating electrodes 401, 402; 402, 403 at a predetermined distance. The spacer may be a fixed tube fixed on the inner cylinder shaft 35. The material of the fixed tube can be polymer such as polyurethane, polyamide, polyimide, fluoropolymer, etc., but the material is not particularly limited thereto as long as the fixed tube has certain rigidity and flexibility. The thickness of the fixed tube is preferably 0.2 mm or more and 1.0 mm or less, for example, in consideration of the external diameter of the heating electrodes 40.

The length DA of the area in which the multiple heating electrodes 40 are located on the inner cylinder shaft 35 is preferably half or more than half the length DC of the inner cylinder shaft 35 in the balloon 25. This allows the liquid in the balloon 25 to be uniformly heated along the longitudinal direction LD.

In the example shown in Figs. 1 to 4, the number of the heating electrodes 40 is three, but the number is not limited thereto. The number of the heating electrodes 40 can be two or more. In addition, in the example shown in Figs. 1 to 4, although the length DD of each heating electrode 40 is 6 mm, the length DD is not limited thereto. The length of each heating electrode 40 may be optional. In addition, in the example shown in Figs. 1 to 4, the distance DB between the adjacent heating electrodes 40 is 4 mm, but the distance DB is not limited thereto. The aforementioned distance DB may be optional. However, when the diameter of the balloon 25 is 25 mm or more and 35 mm or less, in consideration of the length of the area in which the heating electrodes 40 can be located on the inner cylinder shaft 35, and complexity of manufacture and arrangement of the heating electrodes 40, the number of the heating electrodes 40 is preferably 2 or more and 6 or less, the length DD of each heating electrode 40 is preferably 3 mm or more, and the distance DB between the adjacent heating electrodes 40 is preferably 2 mm or more and 14 mm or less. In this specification, when the heating electrodes 401, 402, which are adjacent to each other, are taken by way of example, "the distance DB between the adjacent heating electrodes 40" is the distance between the proximal end of the distally located heating electrode 401 and the distal end of the proximally located heating electrode 402.

In the example shown in Figs. 1 to 4, the distance DY between the proximal end 40b of the multiple heating electrodes 40 (electrode temperature sensor 45) and the distal end 30a of the outer cylinder shaft 30 is 2 mm, but the distance DY is not limited thereto. The distance DY may be optional. However, from the viewpoint of efficiently cool the electrode temperature sensor 45 attached to the proximal end 40b irrespective of the angles at which the balloon 25 is pressed against a target site, the distance DY is preferably 0 mm or more and 5 mm or less. In addition, from the viewpoint of accurately estimating the surface temperature of the balloon 25, the distance DY is preferably 3 mm or more. The reason thereof is described below.

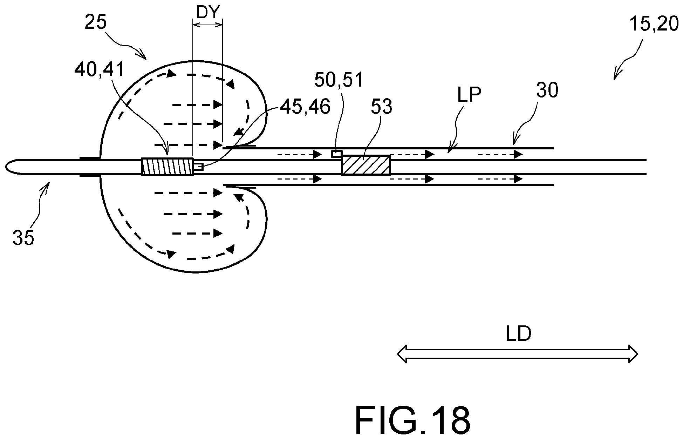

Namely, as described above, the surface temperature of the balloon 25 is estimated based on the information on the liquid temperature in the liquid flow path LP acquired by the liquid-flow-path temperature sensor 50. In order to bring the balloon 25 into tight contact with a target site during ablation, the balloon catheter 15 is sometimes pressed hard against the target site. In this case, since the balloon 25 is so soft that the outer cylinder shaft 30 advances with respect to the inner cylinder shaft 35 distally in the longitudinal direction LD (see Fig. 18). When the distal end 30a of the outer cylinder shaft 30 and the heating electrode 40 on the inner cylinder shaft 35 come too close to each other, the difference between the estimated surface temperature of the balloon 25 and the actual surface temperature thereof becomes large. This is considered to be due to the following reason. Namely, suppose that the distance DY between the distal end 30a of the outer cylinder shaft 30 and the proximal end 40b of the heating electrode 40 is sufficiently large. In this case, when a liquid is discharged from the balloon 25 by the agitator 75, as shown in Fig. 8, the liquid having flown along the surface of the balloon 25 is mainly drawn into the liquid flow path LP. Thus, in this case, the liquid-flow-path temperature sensor 50 can measure a liquid temperature close to the actual surface temperature of the balloon 25. Thus, the estimated surface temperature of the balloon 25, which is estimated based on the information on the temperature of the liquid acquired by the liquid-flow-path temperature sensor 50, has a value close to the actual surface temperature of the balloon 25. On the other hand, suppose that the distance DY between the distal end 30a of the outer cylinder shaft 30 and the proximal end 40b of the heating electrode 40 is excessively small. In this case, as shown in Fig. 18, the liquid having flown along the surface of the balloon 25 is less likely to be drawn into the liquid flow path LP, while the heated liquid around the heating electrode 40 is likely to be directly drawn into the liquid flow path LP. Thus, in the case shown in Fig. 18, the liquid-flow-path temperature sensor 50 will measure a liquid temperature that is significantly higher than the actual surface temperature of the balloon 25. As a result, the estimated surface temperature of the balloon 25 becomes significantly higher than the actual surface temperature. The distance DY is measured with respect to the balloon 25 being inflated with the liquid (see Figs. 2 and 3).

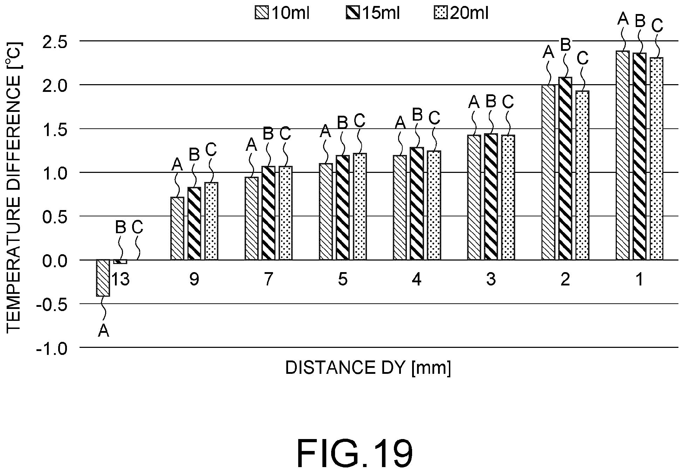

Fig. 19 shows the temperature differences between the estimated surface temperatures and the actual surface temperatures of the balloon 25 due to variation of the distances DY between the heating electrode 40 and the distal end of the outer cylinder shaft 30. The result shown in Fig. 19 was obtained with CAE (computer aided engineering) by simulating temperature distributions of the liquid in the balloon 25 and in the liquid flow path LP near the balloon 25 (near the distal end 30a of the outer cylinder shaft 30). In the examples shown in Fig. 19, the simulations were performed as follows like an actual clinical case. Namely, three models of the balloon catheter system 10 were used, in which the amount of the liquid injected into the balloon 25 was about 10 ml, about 15 ml, and about 20 ml, respectively. In the simulations, each model was controlled such that the temperature of the liquid around the heating electrode 40 became 70°C, and calculation was performed under the condition that the drive power was at 150W.

In Fig. 19, the horizontal axis shows the distance DY between the proximal end 40a of the heating electrode 40 and the distal end 30a of the outer cylinder shaft 30, and the vertical axis shows the temperature difference between the estimated surface temperature and the actual surface temperature of the balloon 25. The estimated surface temperature is a surface temperature of the balloon 25 estimated based on the temperature information acquired by the liquid-flow-path temperature sensor 50. In Fig. 19, the bars A, B, C show the temperature differences for models in which the amount of the liquid injected into the balloon 25 is about 10 ml, about 15 ml, and about 20 ml. As can be understood from Fig. 19, in all models, when the distance DY was 3 mm or more, the temperature difference was less than ±1.5°C. In particular, when the distance DY was 9 mm or more, the temperature difference was less than ±1°C, and when the distance DY was 13 mm or more, the temperature difference was less than ±0.5°C. On the other hand, when the distance DY was 2 mm or less, the temperature difference was significantly greater than the case in which the distance DY was 3 mm or more, i.e., the temperature difference was about 2°C or more. As described with reference to Fig. 18, the reason of the significantly large temperature difference is thought to be that, when the distance DY was 2 mm or less, the liquid heated by the heating electrode 40 was mainly drawn directly to the liquid flow path LP, and the amount of the liquid having passed near the surface of the balloon 25 to flow into the liquid flow path LP was small. The acceptable temperature difference between the estimated surface temperature and the actual surface temperature of the balloon 25 in an actual clinical case is generally less than ±2°C.

Regardless of the difference in angles at which the balloon 25 is pressed against a target site, when the distance DY is sufficiently large, the liquid having flown along the surface of the balloon 25 is likely to flow into the liquid flow path LP upon discharge of a liquid from the balloon 25 to the liquid flow path LP. This allows the surface temperature of the balloon 25 to be accurately determined based on the temperature information of the liquid acquired by the liquid-flow-path temperature sensor 50. This point is described with reference to Fig. 9 and 10. Figs. 9 and 10 show simulation results of temperature distributions of the liquid in the balloon 25 and in the liquid flow path LP near the balloon 25 (near the distal end 30a of the outer cylinder shaft 30) by means of CAE. The simulations shown in Figs. 9 and 10 were performed under the condition that the balloon catheter 15 has the distance DY of 3 mm or more.

Fig. 9 shows the simulation result of a case where the balloon 25 was pressed against a target site, with the inner cylinder shaft 35 and the outer cylinder shaft 30 being in the coaxial state. In the example shown in Fig. 9, the distal end 30a of the outer cylinder shaft 30 and the heating electrode 40 were aligned along the center axis 30X of the outer cylinder shaft 30 at the distal end 30a. In this case, as can be understood from the simulation result of Fig. 9, even when the liquid in the balloon 25 was agitated using the agitator 75, the temperature gradient caused by the location of the heating electrode 40 is generated in the liquid in the balloon 25. When a coil electrode is used as the heating electrode 40, this temperature gradient will show the same tendency as the distribution of current density. In the example shown in Fig. 9, the temperature gradient in the balloon 25 was 5°C or more. However, the liquid temperature in the liquid flow path LP near the distal end 30a of the outer cylinder shaft 30 was substantially equal to the surface temperature of the balloon 25, although it differed from the liquid temperature around the heating electrode 40. Thus, in the example shown in Fig. 9, it can be understood that the surface temperature of the balloon 25 can be detected by measuring the liquid temperature in the liquid flow path LP near the distal end 30a of the outer cylinder shaft 30.

Fig. 10 shows the simulation result of a case where the balloon 25 was pressed against a target site, with the inner cylinder shaft 35 and the outer cylinder shaft 30 being in the non-coaxial state. In the example shown in Fig. 10, the outer cylinder shaft 30 was greatly inclined with respect to the inner cylinder shaft 35 and the heating electrode 40 in the balloon 25. In this case, the distal end 30a of the outer cylinder shaft 30 and the heating electrode 40 were not aligned along the center axis 30X of the outer cylinder shaft 30 at the distal end 30a. Also in this case, as can be understood from the simulation result of Fig. 10, even when the liquid in the balloon 25 was agitated using the agitator 75, the temperature gradient caused by the location of the heating electrode 40 was generated in the balloon 25. Also, in the example shown in Fig. 10, the temperature gradient in the balloon 25 was 5°C or more. However, the liquid temperature in the liquid flow path LP near the distal end 30a of the outer cylinder shaft 30 was substantially equal to the surface temperature of the balloon 25. Thus, also in the example shown in Fig. 10, it can be understood that the surface temperature of the balloon 25 can be detected by measuring the liquid temperature in the liquid flow path LP near the distal end 30a of the outer cylinder shaft 30.

As can be understood from the simulation results of Figs. 9 and 10, depending on the angles at which the balloon 25 is pressed against a target site, the temperature distributions of the liquid in the balloon 25 differs. However, in each case, the temperature of the liquid in the area near the distal end 30a of the outer cylinder shaft 30 and the surface temperature of the balloon 25 were substantially equal to each other. Thus, it can be understood that the surface temperature of the balloon 25 can be detected by measuring the liquid temperature in the liquid flow path LP near the distal end 30a of the outer cylinder shaft 30, regardless of the difference in angles at which the balloon 25 is pressed against a target site.

In summary, when the distance DY between the proximal end 40b of the heating electrode(s) 40 and the distal end 30a of the outer cylinder shaft 30 is 3 mm or more, the surface temperature of the balloon 25 can be accurately detected, irrespective of the angle at which the balloon 25 is pressed against a target site.

The balloon catheter 15 and the balloon catheter system 10 according to this embodiment have been described above with reference to Figs. 1 to 6. However, the structures of the balloon catheter 15 and the balloon catheter system 10 are not limited to the aforementioned ones. The structures of the balloon catheter 15 and the balloon catheter system 10 may be variously modified.

For example, in the above example, the thermosensitive part 46 of the electrode sensor 45 is attached to the proximal end 40b of the heating electrodes 40, but the present invention is not limited thereto. The thermosensitive part 46 of the electrode sensor 45 may be attached to a part other than the proximal end 40b of the heating electrodes 40. Further, the electrode temperature sensor 45 may not be attached to the heating electrodes 40, as long as it can acquire information on the temperature of the heating electrodes 40. In other words, the thermosensitive part 46 of the electrode temperature sensor 45 may not be in contact with the heating electrodes 40. The thermosensitive part 46 may be provided near the heating electrodes 40 but separated from the heating electrodes 40. In this case, the electrode temperature sensor 45 can measure the temperature of the liquid near the heating electrodes 40. The temperature of the liquid near the heating electrodes 40 is substantially equal to the temperature of the heating electrodes 40. Thus, although the thermosensitive part 46 is separated from the heating electrodes 40, information on the temperature of the heating electrodes 40 can be acquired by the electrode temperature sensor 45. The balloon catheter 15 in this embodiment allows the liquid supplied from the liquid flow path LP to the balloon 25 to flow in the balloon 25 along the heating electrodes 40 on the inner cylinder shaft 35, either when the balloon 25 is pressed against a target site in the axial state or when the balloon 25 is pressed against a target site in the non-coaxial state. As a result, in both cases, the heated liquid around the heating electrodes 40 can be efficiently diffused by the aforementioned liquid to cool the heating electrodes 40. In such a balloon catheter 15, either when the balloon 25 is pressed against a target site in the axial state or when the balloon 25 is pressed against a target site in the non-coaxial state, the electrode temperature sensor 45 is cooled by the liquid supplied from the liquid flow path LP to the balloon 25, as long as the electrode temperature sensor 45 is located to be capable of measuring the temperature of the heating electrodes 40 or the liquid near the heating electrodes 40. Thus, the amount of electric energy supplied from the control unit 70 to the heating electrodes 40 is maintained during ablation.

In the illustrated example, one electrode temperature sensor 45 is provided for the multiple heating electrodes 40, but the present invention is not limited thereto. The balloon catheter 15 may include multiple electrode temperature sensors 45. In this case, one electrode temperature sensor 45 may be provided on one heating electrode 40. In other words, the balloon catheter 15 may have the electrode temperature sensors 45 whose number is the same as that of the heating electrodes 40.

Further, in the balloon catheter 15, a liquid flow path through which the liquid discharged from the inner space of the balloon 25 flows may be provided independently of the liquid flow path LP through which the liquid to be supplied into the inner space of the balloon 25 flows. For example, the lumen as the inner space of the inner cylinder shaft 35 may be used as the liquid flow path through which the liquid discharged from the inner space of the balloon 25 flows.

As described above, when the supply liquid flow path LP through which the liquid to be supplied into the inner space of the balloon 25 flows and the discharge liquid flow path through which the liquid discharged from the inner space of the balloon 25 flows differ from each other, the catheter system 10 may have a discharge unit that discharges a liquid from the discharge liquid flow path, separately from the supply unit 74 that supplies a liquid to the supply liquid flow path LP.

In addition, when the supply liquid flow path LP through which the liquid to be supplied into the inner space of the balloon 25 flows and the discharge liquid flow path through which the liquid discharged from the inner space of the balloon 25 flows differ from each other, the agitator 75 may supply a liquid to the supply liquid flow path LP and discharge a liquid from the discharge liquid flow path. In this case, the liquid in the balloon 25 is agitated by circulating the liquid through a flow path constituted by the supply liquid flow path LP, the inner space of the balloon 25, and the discharge liquid flow path. In this case, the liquid-flow-path temperature sensor 50 is provided in the discharge liquid flow path, through which the liquid discharged from the inner space of the balloon 25 flows.

### EXAMPLES

Some examples included in the aforementioned embodiment will be described in comparison with a comparative example. In the drawings used in the below description of the examples and the description of the comparative example, the same symbols as those used for the corresponding parts in the aforementioned description will be used for parts that can be configured in the same way as in the aforementioned description, and duplicate description will be omitted.

### <Example 1>

In order to produce the balloon catheter 15 (see Figs. 2 to 4) in Example 1, a polyurethan balloon 25 having a diameter of 30 mm and a thickness of 20 µm was blow molded.

A polyurethan tube having an external diameter of 3.6 mm, an internal diameter of 3.0 mm, and an entire length of 1000 mm was used as the outer cylinder shaft 30. A polyamide tube having an external diameter of 1.6 mm, an internal diameter of 1.2 mm, and an entire length of 1100 mm was used as the inner cylinder shaft 35.

Three coil electrodes having a length DD of 6 mm were manufactured as the heating electrodes 40 by stripping part of the electrically insulating protective coats provided on the leas wire 42 of the heating electrodes 40 and the lead wire 47 of the electrode temperature sensor 45, and by winding the lead wire 42 around the inner cylinder shaft 35 with sandwiching the lead wire 47. The distance DB between the adjacent heating electrodes 40 was 4 mm. The lead wire 42 and the lead wire 52 are welded and fixed on the inner cylinder shaft 35 to form a thermocouple as the liquid-flow-path temperature sensor 50.

The inner cylinder shaft 35 was slidably inserted to the lumen of the outer cylinder shaft 30. The distal end 25a of the balloon 25 was fixed on the distal end 35a of the inner cylinder shaft 35. The distal end 30a of the outer cylinder shaft 30 was inserted from the proximal end 25b of the balloon 25 into the balloon 25, and the proximal end 25b of the balloon 25 was fixed on the outer cylinder shaft 30 at a position more proximal than the distal end 30a.

The polycarbonate handle 60 was provided on the rear ends of the outer cylinder shaft 30 and the inner cylinder shaft 35. The handle 60 was constituted by the first handle part (front handle part) 61 connected to the outer cylinder shaft 30, and the second handle part (rear handle part) 62 connected to the inner cylinder shaft 35. The shape of the balloon 25 could be deformed by a sliding operation of the second handle part 62 with respect to the first handle part 61 to slide the inner cylinder shaft 35 inside the outer cylinder shaft 30.

### <Example 2>

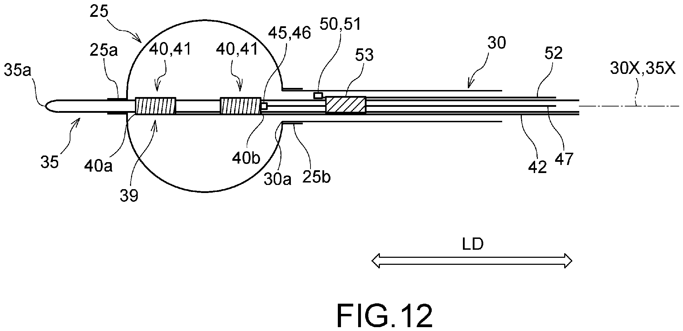

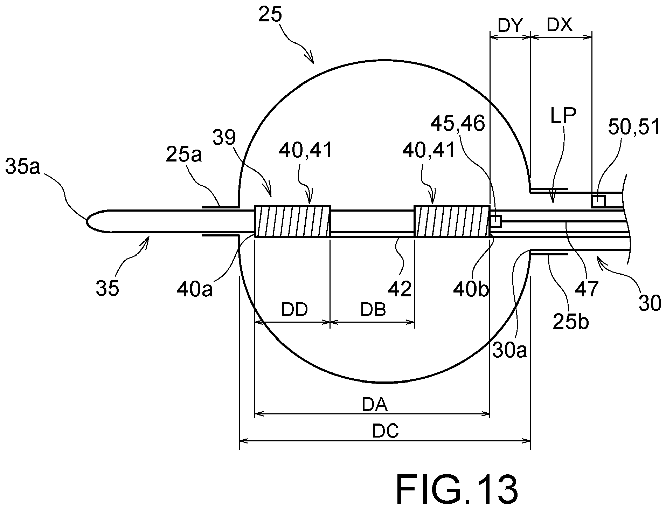

The balloon catheter 15 (see Figs. 12 and 13) in Example 2 was produced similarly to the balloon catheter 15 in Example 1, except that, in Example 2, two coil electrodes having the length DD of 6 mm were produced as the heating electrodes 40 and that the distance DB between the adjacent heating electrodes 40 was 14 mm.

### <Example 3>

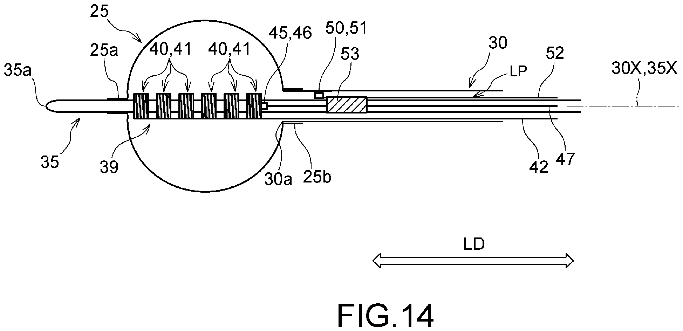

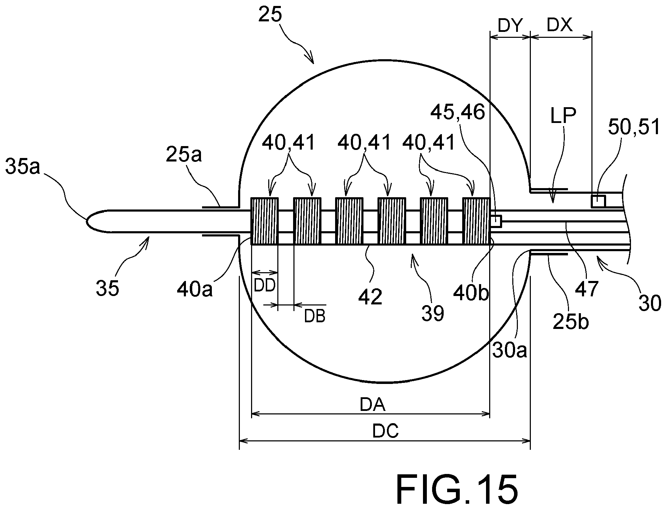

The balloon catheter 15 (see Figs. 14 and 15) in Example 3 was produced similarly to the balloon catheter 15 in Example 1, except that, in Example 3, six coil electrodes having the length DD of 3 mm were produced as the heating electrodes 40 and that the distance DB between the adjacent heating electrodes 40 was 2 mm.

### <Comparative Example>

The balloon catheter 15 (see Fig. 16) in Comparative Example was produced similarly to the balloon catheter 15 in Example 1, except that, in Comparative Example, one coil electrode having the length DD of 13 mm was produced as the heating electrode 40.

### <Comparative Experiments of Balloon Surface Temperature>

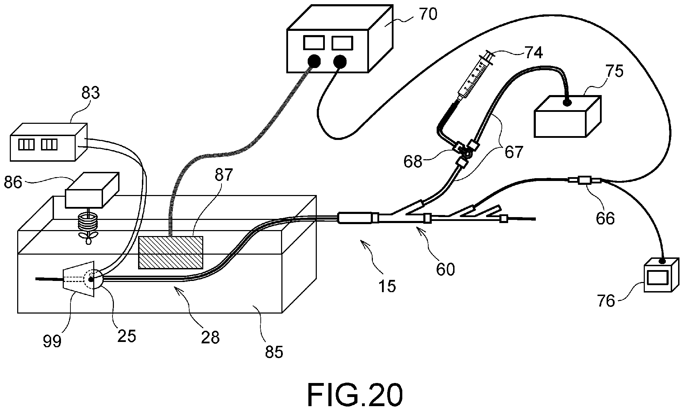

As shown in Fig. 20, the balloon catheters 15 of Examples 1-3 and Comparative Example were used to apply ablation therapy to a pseudo-living body 99 that imitated the left atrial pulmonary vein opening of the human body. The pseudo-living body 99 was made by molding an acrylic resin and was immersed in physiological saline solution held in a water tank 85. During the experiment, a pipe heater 86 with an agitator was used to agitate the physiological saline solution in the water tank 85, and the temperature of the physiological saline solution was maintained at 36.5°C. A counter electrode 87, which generates a high-frequency current between it and the high-frequency current conduction electrode(s) 41 of the catheter body 20, was located on a sidewall of the water tank 85. The physiological saline solution in the water tank 85 was a solution of 0.9 wt% salt (sodium chloride) dissolved in water.

The liquid supplied from the supply unit 74 into the liquid flow path LP and the balloon 25 was a mixture of a contrast agent for x-ray contrast and a physiological saline solution with 0.9 wt% salt (sodium chloride) dissolved in water. The amount of a liquid injected into the balloon 25 was set at two levels, 10 mL and 20 mL, which are commonly used in actual ablation treatments. The contrast agent mixed in the liquid was Omnipark (registered trademark) manufactured by Daiichi Sankyo Company, Ltd. The dilution ratio of the contrast agent was set at two levels, 1:2 and 1:3. The dilution ratio of the contrast agent means "volume of saline solution : volume of contrast agent".

The surface temperature of each balloon 25, when the balloon 25 was pressed perpendicularly against the opening surface of the pulmonary vein opening of the pseudo-living body 99 (i.e., when the balloon 25 was pressed against the opening surface in the coaxial state), was measured by using a measurement unit 83. In addition, the surface temperature of each balloon 25, when the balloon 25 was pressed against the opening surface of the pulmonary vein opening of the pseudo-living body 99 at an angle between 45 degrees and 60 degrees (i.e., when the balloon 25 was pressed against the opening surface in the non-coaxial state, more specifically, when an angle between a normal of the opening surface and the center axis 30X of the outer cylinder shaft 30 at the distal end 30a of the outer cylinder shaft 30 was between 30 degrees and 45 degrees), was measured by using the measurement unit 83.

The results of the comparative experiments are described below.

### <<Result of Comparative Example>>

### (Case of Coaxial State)

In the balloon catheter 15 in Comparative Example, in the case of the coaxial state (see Fig. 16), the orientation of the distal end 30a of the outer cylinder shaft 30 followed the direction in which the inner cylinder shaft 35 extended in the balloon 25. In this case, when the set electrode temperature of the heating electrode 40 was 70°C, the surface temperature of the balloon 25 was 65.6°C.

### (Case of Non-Coaxial State)

On the other hand, in the case of the non-coaxial state (see Fig. 17), the inner cylinder shaft 35 was curved along the outer cylinder haft 30 in the outer cylinder shaft 30. However, the inner cylinder shaft 35 was not almost curved in the balloon 25. The orientation of the distal end 30a of the outer cylinder shaft 30 deviated from the direction in which the inner cylinder shaft 35 extended in the balloon 25. In this case, when the set electrode temperature of the heating electrode 40 was 70°C, the surface temperature of the balloon 25 was 58.4°C.

### <<Result of Example 1>>

### (Case of Coaxial State)

In the balloon catheter 15 in Example 1 (see Figs. 2 to 4), in the case of the coaxial state, the orientation of the distal end 30a of the outer cylinder shaft 30 followed the direction in which the inner cylinder shaft 35 extended in the balloon 25. In this case, when the set electrode temperature of the heating electrodes 40 was 70°C, the surface temperature of the balloon 25 was 71.8°C.

### (Case of Non-Coaxial State)

On the other hand, in the case of the non-coaxial state, the inner cylinder shaft 35 was smoothly curved from the inside of the balloon 25 to the inside of the outer cylinder shaft 30. The orientation of the distal end 30a of the outer cylinder shaft 30 followed the curve of the inner cylinder shaft 35 in the balloon 25. In this case, when the set electrode temperature of the heating electrodes 40 was 70°C, the surface temperature of the balloon 25 was 71.6°C.

### <<Result of Example 2>>

### (Case of Coaxial State)

In the balloon catheter 15 in Example 2 (see Figs. 12 and 13), in the case of the coaxial state, the orientation of the distal end 30a of the outer cylinder shaft 30 followed the direction in which the inner cylinder shaft 35 extended in the balloon 25. In this case, when the set electrode temperature of the heating electrodes 40 was 70°C, the surface temperature of the balloon 25 was 72.6°C.

### (Case of Non-Coaxial State)

On the other hand, in the case of the non-coaxial state, the inner cylinder shaft 35 was smoothly curved from the inside of the balloon 25 to the inside of the outer cylinder shaft 30. The orientation of the distal end 30a of the outer cylinder shaft 30 followed the curve of the inner cylinder shaft 35 in the balloon 25. In this case, when the set electrode temperature of the heating electrodes 40 was 70°C, the surface temperature of the balloon 25 was 72.2°C.

### <<Result of Example 3>>

### (Case of Coaxial State)

In the balloon catheter 15 in Example 3 (see Figs. 14 and 15), in the case of the coaxial state, the orientation of the distal end 30a of the outer cylinder shaft 30 followed the direction in which the inner cylinder shaft 35 extended in the balloon 25. In this case, when the set electrode temperature of the heating electrodes 40 was 70°C, the surface temperature of the balloon 25 was 70.9°C.

### (Case of Non-Coaxial State)

On the other hand, in the case of the non-coaxial state, the inner cylinder shaft 35 was smoothly curved from the inside of the balloon 25 to the inside of the outer cylinder shaft 30. The orientation of the distal end 30a of the outer cylinder shaft 30 followed the curve of the inner cylinder shaft 35 in the balloon 25. In this case, when the set electrode temperature of the heating electrodes 40 was 70°C, the surface temperature of the balloon 25 was 70.5°C.

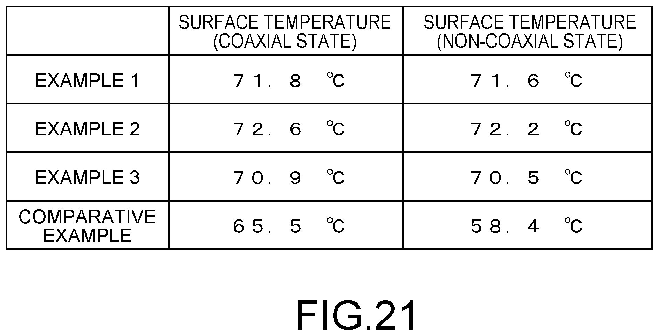

Fig. 21 shows the results of the aforementioned comparative experiments. As can be understood from Fig. 21, no remarkable difference in the surface temperature of the balloon 25 was found between the case in which the balloon 25 was pressed against the pseudo-living body 99 in the coaxial state and the case in which the balloon 25 was pressed thereagaint in the non-coaxial sate. On the other hand, in the comparative example, temperature difference of about 7°C in the surface temperature of the balloon 25 occurred between the case in which the balloon 25 was pressed against the pseudo-living body 99 in the coaxial state and the case in which the balloon 25 was pressed thereagaint in the non-coaxial sate. In addition, even though the experiments in Examples 1 to 3 and Comparative Examples were all carried out such that the set electrode temperature of the heating electrode 40 was 70°C, the surface temperature of the balloon 25 in Comparative Example was lower than the surface temperatures of the balloons in Examples 1 to 3 by 5°C or more. Such a difference is thought to be caused for the following reasons. Namely, in Examples 1 to 3, since the liquid supplied from the liquid flow path LP into the balloon 25 flowed along the heating electrode 40 on the inner cylinder shaft 35, the heated liquid around the heating electrodes 40 could be efficiently diffused, and the electrode temperature sensor 45 could be efficiently cooled by the aforementioned liquid. As a result, the surface temperature of the balloon 25 could be efficiently increased, and the amount of electric energy supplied from the control unit 70 to the heating electrodes 40 was maintained. On the other hand, in the case of Comparative Example, since the flow of the liquid supplied from the liquid flow path LP to the balloon 25 deviated from the heating electrode 40 on the inner cylinder shaft 35, the heated liquid around the heating electrode 40 could not be efficiently diffused, and the electrode temperature sensor 45 could not be efficiently cooled by the aforementioned liquid. Thus, in Comparative Example, the surface temperature of the balloon 25 could not be increased to the same extent as in Examples 1 to 3. Since the electrode temperature sensor 45 continued to detect a high temperature, the amount of electric energy supplied from the control unit 70 to the heating electrode 40 was decreased.

According to this embodiment, the balloon catheter 15 comprises the balloon 25, the catheter shaft 28, the heating unit 39, and the electrode temperature sensor 45. The catheter shaft 28 has the outer cylinder shaft 30 connected to the proximal end 25b of the balloon 25, and the inner cylinder shaft 35 extending into the balloon 25 to be connected to the distal end 25a of the balloon 25. The inner cylinder shaft 35 passes inside the outer cylinder shaft 30. The gap between the inner cylinder shaft 35 and the outer cylinder shaft 30 defines the liquid flow path LP leading to the inner space of the balloon 25. The heating unit 39 includes multiple the heating electrodes 40. The multiple heating electrodes 40 are distributed over the outer circumferential surface of the inner cylinder shaft 35. The electrode temperature sensor 45 is provided in the balloon 25 to acquire information on the temperature of the heating electrodes 40.

According to such a balloon catheter 15, when the balloon 25 is pressed against a target site in the non-coaxial state, the inner cylinder shaft 35 can be curved along the flow of the liquid supplied from the liquid flow path LP into the balloon 25 through the distal end 30a of the outer cylinder shaft 30. Thus, either when the balloon 25 is pressed against a target site in the coaxial state or when the balloon 25 is pressed thereagainst in the non-coaxial state, the liquid supplied from the liquid flow path LP to the balloon 25 can flow in the balloon 25 along the heating electrodes 40 on the inner cylinder shaft 35 to efficiently diffuse the heated liquid around the heating electrodes 40. This allows the surface temperature of the balloon 25 to be efficiently increased. In addition, since the liquid supplied from the liquid flow path LP to the balloon 25 flows along the heating electrodes 40, the heating electrodes 40 and the electrode temperature sensor 45 can be cooled by the aforementioned liquid. As a result, the amount of electric energy supplied from the control unit 70 to the heating electrodes is maintained, so that the liquid heating effect by the heating electrodes 40 and the counter electrode 77 is maintained. As a result, no remarkable difference in the surface temperature of the balloon 25 occurs between when the balloon 25 is pressed against a target site in the coaxial state and when the balloon 25 is pressed thereagainst in the non-coaxial state, thereby reliable control of the surface temperature of the balloon 25 is achieved. In addition, it is easy to increase the surface temperature of the balloon 25 up to a desired temperature.

In addition, in the balloon catheter 15 in this embodiment, the heating electrodes 40 are coil electrodes that are formed by winding the single lead wire 42 around the inner cylinder shaft 35 at multiple locations thereon. This can avoid locating of multiple lead wires 42 in the liquid flow path LP. Thus, decrease in space in the liquid flow path LP and increase in diameter of the outer cylinder shaft 30, which might be caused by multiple lead wires 42, can be avoided.

In addition, in the balloon catheter according to this embodiment, the length DA of the area in which the heating electrodes 40 are located is half or more than half the length DC of the inner cylinder shaft 35 in the balloon. This allows the liquid in the balloon 25 to be uniformly heated along the longitudinal direction LD.

In addition, in the balloon catheter 15 according to this embodiment, the number of heating electrodes 40 is 2 or more and 6 or less.

In addition, in the balloon catheter 15 according to this embodiment, the length of the heating electrode 40 is 3 mm or more. This facilitates manufacture of the heating electrodes 40.

In addition, in the balloon catheter 15 according to this embodiment, the distance DB between the heating electrode 40 and the heating electrode(s) 40 adjacent thereto is 2 mm or more and 14 mm or less.

In addition, in the balloon catheter 15 according to this embodiment, the distance DY between the proximal end 40a of the multiple heating electrodes 40 and the distal end 30a of the outer cylinder shaft 30 is 0 mm or more and 5 mm or less. Thus, the most proximally located heating electrode 40 of the multiple electrodes 40 and the electrode temperature sensor 45 corresponding thereto can be efficiently cooled by the liquid supplied from the liquid flow path LP into the balloon 25. This contributes to maintaining the amount of electric energy supplied from the control unit 70 to the heating electrodes 40.

In addition, the balloon catheter 15 according to this embodiment comprises the liquid-flow-path temperature sensor 50 that is provided in the liquid flow path LP to acquire information on the temperature of the liquid flowing in the liquid flow path. In addition, the distance DY between the proximal end 40a of the multiple heating electrodes 40 and the distal end 30a of the outer cylinder shaft 30 is 3 mm or more and 5 mm or less. Thus, the surface temperature of the balloon 25 can be accurately estimated based on the information acquired by the liquid-flow-path temperature sensor 50. As a result, the surface temperature of the balloon 25 can be more accurately controlled.

In addition, the balloon catheter system 10 according to this embodiment comprises the aforementioned balloon catheter 10, the supply unit 74 that supplies a liquid to the liquid flow path LP, the agitator 75 that agitates the liquid in the balloon by repeatedly supplying a liquid to the liquid flow path LP and discharging a liquid from the liquid flow path LP, and the control unit 70 electrically connected to the heating electrodes 40 to provide the heating electrodes with electric energy. The control unit 70 provides the heating electrodes 40 with electric energy based on the information on the temperature acquired by the electrode temperature sensor 45.

According to such a balloon catheter system 10, when the balloon 25 is pressed against a target site in the non-coaxial state, the inner cylinder shaft 35 can be curved along the flow of the liquid supplied from the liquid flow path LP into the balloon 25 through the distal end 30a of the outer cylinder shaft 30. Thus, either when the balloon 25 is pressed against a target site in the coaxial state or when the balloon 25 is pressed thereagainst in the non-coaxial state, the liquid supplied from the liquid flow path LP to the balloon 25 can flow in the balloon 25 along the heating electrodes 40 on the inner cylinder shaft 35 to efficiently diffuse the heated liquid around the heating electrodes 40. This allows the surface temperature of the balloon 25 to be efficiently increased. In addition, since the liquid supplied from the liquid flow path LP to the balloon 25 flows along the heating electrodes 40, the heating electrodes 40 and the electrode temperature sensor 45 can be cooled by the aforementioned liquid. As a result, the amount of electric energy supplied from the control unit 70 to the heating electrodes is maintained, so that the liquid heating effect by the heating electrodes 40 and the counter electrode 77 is maintained. As a result, no remarkable difference in the surface temperature of the balloon 25 occurs between when the balloon 25 is pressed against a target site in the coaxial state and when the balloon 25 is pressed thereagainst in the non-coaxial state, thereby reliable control of the surface temperature of the balloon 25 is achieved. In addition, it is easy to increase the surface temperature of the balloon 25 up to a desired temperature.

In the balloon catheter system 10 according to this embodiment, the balloon catheter 15 has the liquid-flow-path temperature sensor 50 provided in the liquid flow path LP to acquire information on the temperature of the liquid flowing in the liquid flow path. In addition, the distance between the proximal end 40a of the multiple heating electrodes 40 and the distal end 30a of the outer cylinder shaft 30 is 3 mm or more and 5 mm or less. In addition, the distance from the distal end 30a of the outer cylinder shaft 30 up to the liquid-flow-path temperature sensor 50 is no more than the value obtained by dividing the amount of a liquid discharged by the agitator 75 from the liquid flow path LP by a cross-sectional area of the liquid flow path LP.

According to such a balloon catheter system 10, the surface temperature of the balloon 25 can be accurately estimated based on the information acquired by the liquid-flow-path temperature sensor 50. As a result, the surface temperature of the balloon 25 can be more accurately controlled.

The aforementioned embodiment and its variations are included in the scope and gist of the invention, as well as in the invention described in the claims and its equivalents. It is also possible, of course, to combine partly the above-described embodiment and its variations as appropriate, within the scope of the gist of the invention.

10 ··· Catheter system, 15 ... Balloon catheter, 25 ... Balloon, 30 ... Outer cylinder shaft, 30X ··· Cetner axis of outer cylinder shaft 30 ... Inner cylinder shaft, 35X ··· Center axis of inner cylinder shaft, 40 ... Heating electrode, 45 ... Electrode temperature sensor, 50 ... Liquid-flow-path temperature sensor, 60 ··· Handle, 70 ... Control unit, 75 ... Agitator, LP ... Liquid flow path

## Claims

1. A balloon catheter comprising a balloon, a catheter shaft, a heating unit, and an electrode temperature sensor,
wherein:
the catheter shaft has an outer cylinder shaft connected to a proximal end of the balloon, and an inner cylinder shaft extending into the balloon to be connected to a distal end of the balloon, the inner cylinder shaft passes inside the outer cylinder shaft, and a gap between the inner cylinder shaft and the outer cylinder shaft defines a liquid flow path leading to an inner space of the balloon;
the heating unit includes multiple heating electrodes;
the multiple heating electrodes are distributed over an outer circumferential surface of the inner cylinder shaft in the balloon; and
the electrode temperature sensor is provided in the balloon to acquire information on a temperature of the heating electrodes.

2. The balloon catheter according to claim 1, wherein
the heating electrodes are coil electrodes that are formed by winding a single lead wire around the inner cylinder shaft at multiple locations thereon.

3. The balloon catheter according to claim 1 or 2, wherein
a length of an area in which the heating electrodes are located is half or more than half a length of the inner cylinder shaft in the balloon.

4. The balloon catheter according to any one of claims 1 to 3, wherein
the number of heating electrodes is between 2 and 6.

5. The balloon catheter according to any one of claims 1 to 4, wherein
a length of the heating electrode is 3 mm or more.

6. The balloon catheter according to any one of claims 1 to 5, wherein
a length between the heating electrode and the heating electrode adjacent thereto is between 2 and 14 mm.

7. The balloon catheter according to any one of claims 1 to 6, wherein
a distance between a proximal end of the multiple heating electrodes and a distal end of the outer cylinder shaft is between 0 and 5 mm.

8. The balloon catheter according to claim 7, comprising a liquid-flow-path temperature sensor provided in the liquid flow path to acquire information on a temperature of a liquid flowing in the liquid flow path,
wherein the distance between the proximal end of the multiple heating electrodes and the distal end of the outer cylinder shaft is between 3 and 5 mm.

9. A balloon catheter system comprising:
a balloon catheter according to any one of claims 1 to 8;
a supply unit that supplies a liquid to the liquid flow path;
an agitator that agitates a liquid in the balloon by repeatedly supplying a liquid to the liquid flow path and discharging a liquid from the liquid flow path; and
a control unit electrically connected to the heating electrodes to provide the heating electrodes with electric energy;
wherein the control unit provides the heating electrodes with electric energy based on information on a temperature acquired by the electrode temperature sensor.

10. The balloon catheter system according to claim 9, wherein:
the balloon catheter has a liquid-flow-path temperature sensor provided in the liquid flow path to acquire information on a temperature of a liquid flowing in the liquid flow path;
the distance between the proximal end of the multiple heating electrodes and the distal end of the outer cylinder shaft is between 3 and 5 mm; and
a distance from the distal end of the outer cylinder shaft up to the liquid-flow-path temperature sensor is no more than a value obtained by dividing an amount of the liquid discharged by the agitator from the liquid flow path by a cross-sectional area of the liquid flow path.
